# EUROPEAN PATENT SPECIFICATION

(11) **EP 4 151 671 B1**
(45) Date of publication and mention of the grant of the patent: **30.04.2025**
(21) Application number: 21803803.2
(22) Date of filing: 07.05.2021
(51) Int. Cl.: C08G 71/04, C07C 68/06, C07C 69/96

(54) **METHOD FOR PRODUCING POLYURETHANE**
VERFAHREN ZUR HERSTELLUNG VON POLYURETHAN
PROCÉDÉ DE FABRICATION DE POLYURÉTHANE

(30) Priority: 11.05.2020 JP 2020083148
(43) Date of publication of application: 22.03.2023
(73) Proprietor: National University Corporation Kobe University, Kobe-shi, Hyogo 657-8501 (JP); AGC INC., Chiyoda-ku, Tokyo 1008405 (JP)
(72) Inventor: TSUDA, Akihiko, Kobe-shi, Hyogo 657-8501 (JP); OKAZOE, Takashi, Tokyo 100-8405 (JP); WADA, Hiroshi, Tokyo 100-8405 (JP); TANAKA, Hideaki, Tokyo 100-8405 (JP); SUNAYAMA, Yoshitaka, Tokyo 100-8405 (JP); KAKIUCHI, Toshifumi, Tokyo 100-8405 (JP)
(74) Representative: Müller-Boré & Partner Patentanwälte PartG mbB
(86) International application number: PCT/JP2021/017511
(87) International publication number: WO 2021/230151

(56) References cited:
- WO-A1-2014/171367
- WO-A1-2017/104709
- WO-A1-2018/211953
- WO-A1-2020/100970
- JP-A- 2017 530 091

## Description

### TECHNICAL FIELD

The present invention relates to a method for efficiently producing a high quality polyurethane.

### BACKGROUND ART

Polyurethane can be used as a raw material of resin and fiber having high stretchability due to excellent flexibility and elasticity. On the one hand, polyurethane can become also hard and tough by changing the chemical structure. In addition, polyurethane is very superior material due to excellent low temperature property, wear resistance, impact resistance, oil resistance or the like.

Polyurethane is generally produced by reacting a diisocyanate compound with a divalent alcohol compound. Such an isocyanate compound must be preserved under strict conditions such as low humidity and low temperature, since an isocyanate compound is toxic, is highly reactive and is easily reacted with water. Polyurethane can be also produced by using phosgene in place of an isocyanate compound, and an isocyanate compound itself is industrially produced by reacting a primary amine compound with phosgene (Patent document 1 or the like). Phosgene is a toxic compound. For example, phosgene is easily reacted with water to produce hydrogen chloride and has a history of being used as a poison gas. In addition, when a compound is produced by using phosgene, a chlorine component remains in the compound. For example, it is described in Non-patent document 1 that a polycarbonate produced from phosgene contains several dozen ppm to several hundred ppm of a chloride compound, even if the polycarbonate is exhaustively purified. A chlorine component also remains in polyurethane produced from phosgene, and the remaining chlorine component causes yellowing of polyurethane and adversely affects metals and living organisms. Thus, technology to produce polyurethane without using isocyanate compounds and phosgene has been studied.

For example, Patent document 2 and Non-patent documents 2 and 3 disclose a method for producing polyurethane by reacting a diphenyl carbonate optionally being substituted by a nitro group and a fluoro group with a diamino compound. However, a diphenyl carbonate itself is generally synthesized by reacting phosgene and phenol, and a method for synthesizing a diphenyl carbonate without using phosgene requires many steps (Non-patent document 4). On the one hand, the inventors of the present invention have developed a method for safely and efficiently producing a carbonate derivative such as diphenyl carbonate (Patent documents 3 and 4).

Patent document 5 discloses a biscarbonate compound optionally having a fluoro group as a non-aqueous medium for a non-aqueous electrolyte of a secondary battery.

### PRIOR ART DOCUMENT

### PATENT DOCUMENT

Patent document 1: WO 2017/104709
Patent document 2: US 9062160 B
Patent document 3: WO 2018/211952
Patent document 4: WO 2018/017349
Patent document 5: JP H10-149840 A

### NON-PATENT DOCUMENT

Non-patent document 1: FUKUOKA Shinsuke et al., Polymer Journal, Vol. 39, No. 2, pp. 91-114 (2007)
Non-patent document 2: Daniela M. Fidalgo et al., Journal of Polymer Science Part A, 2013, 51, pp. 463-470
Non-patent document 3: Amaury Bossion et al., Langmuir, 2017, 33, pp. 1959-1968
Non-patent document 4: KOMIYA Kyosuke, MASAMOTO Junzo, Journal of Japan Society for Production, Vol. 11, No. 2, pp. 109-114 (2005)

### DISCLOSURE OF THE INVENTION

### PROBLEMS TO BE SOLVED BY THE INVENTION

The technology to produce polyurethane by using diphenyl carbonate without using an isocyanate compound nor phosgene has been already developed as described above. The inventors of the present invention, however, found that phenol is produced as a by-product in the above-described reaction, the phenol cannot be completely removed from the biscarbonate compound and the polyurethane and thus remains in the polyurethane, and the remaining phenol is easily oxidized to cause deterioration of the quality of the polyurethane. For example, the remaining phenol causes the coloration and the decrease of the polymerization degree of the polyurethane. In addition, the inventors experimentally found that the remaining phenol inhibits a polymerization reaction.

Thus, the objective of the present invention is to provide a method for efficiently producing a high quality polyurethane.

### MEANS FOR SOLVING THE PROBLEMS

The inventors of the present invention repeated intensive studies in order to solve the above-described problems. As a result, the inventors completed the present invention by finding that an amount of the remaining fluoroalcohol compound as a by-product can be reduced by using the specific fluorocarbonate compound as a raw material to produce polyurethane, and even if the fluoroalcohol compound remains, the remaining fluoroalcohol compound surprisingly gives preferred properties to the polyurethane.

The present invention is hereinafter described.

[1] A method for producing polyurethane, the method comprising the steps of:
   reacting a fluorocarbonate compound represented by the following formula (I) and a divalent alcohol compound represented by the following formula (II) to obtain a biscarbonate compound represented by the following formula (III), 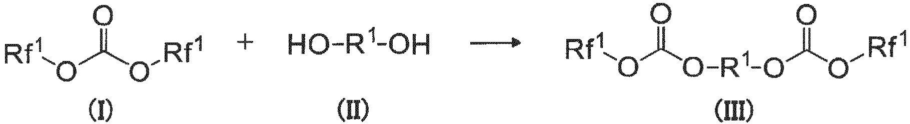 wherein
   Rf¹ is independently an aliphatic hydrocarbon group having a fluoro group,
   R¹ is a divalent organic group,
   and reacting the biscarbonate compound represented by the above formula (III) and a divalent amino compound represented by the following formula (IV) to obtain a polyurethane represented by the following formula (V), 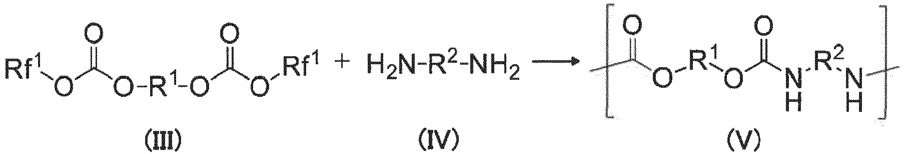 wherein
      Rf¹ and R¹ are the same as the above,
      R² is a divalent organic group.
[2] The method according to the above [1], wherein the fluorocarbonate compound represented by the formula (I) and the divalent alcohol compound represented by the formula (II) are reacted in the presence of a base.
[3] The method according to the above [1] or [2], wherein R¹ is a C₂₋₁₀ alkanediyl group optionally substituted by a halogeno group.
[4] The method according to the above [1] or [2], wherein R¹ is the divalent organic group represented by the formula -R³-[-X-R³-]ₘ-, wherein X is O or S, R³ is a C₁₋₈ alkanediyl group optionally substituted by a halogeno group, and m is an integer of 1 or more and 180 or less.
[5] The method according to any one of the above [1] to [4], wherein R² is a C₂₋₁₀ alkanediyl group optionally having a halogeno group.
[6] The method according to any one of the above [1] to [4], wherein R² is a C₁₋₆ alkanediyl - C₆₋₁₂ aryldiyl - C₁₋₆ alkanediyl group.
[7] A biscarbonate compound represented by the following formula (III-1): 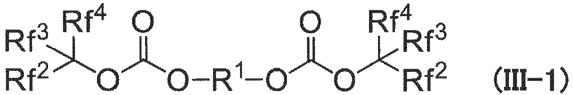 wherein
   Rf² is H or an aliphatic hydrocarbon group having a fluoro group,
   Rf³ and Rf⁴ are independently an aliphatic hydrocarbon group having a fluoro group,
   R¹ is a divalent organic group.

### EFFECT OF THE INVENTION

Polyurethane can be produced without using a toxic isocyanate compound by the present invention method. In addition, diphenyl carbonate as the alternative of phosgene is also not needed to be used, and thus phenol, which is a by-product derived from diphenyl carbonate and causes the coloration and the decrease of the polymerization degree of polyurethane, does not remain. A fluoroalcohol may remain as alternated; however, an amount of the remaining fluoroalcohol is smaller than an amount of the remaining phenol in the case where diphenyl carbonate is used, and the remaining fluoroalcohol may give preferred properties to polyurethane. Thus, the present invention is industrially very useful as the technology to safely and efficiently produce a high quality polyurethane.

### MODE FOR CARRYING OUT THE INVENTION

The method for producing polyurethane according to the present invention comprises the step of reacting a fluorocarbonate compound represented by the formula (I) and a divalent alcohol compound represented by the formula (II) to obtain a biscarbonate compound represented by the formula (III), and the step of reacting the biscarbonate compound represented by the formula (III) and a divalent amino compound represented by the formula (IV) to obtain a polyurethane represented by the formula (V). Hereinafter, each step is described, and the present invention is not restricted to the following specific examples. The "compound represented by the formula (x)" is hereinafter abbreviated as the "compound (x)".

### 1. Step to produce biscarbonate compound

The fluorocarbonate compound (I) and the divalent alcohol compound (II) are reacted to obtain the biscarbonate compound (III) in this step. The desired properties such as high strength, flexibility and water repellency can be given to polyurethane due to the combination of R² of the divalent amino compound (IV) and R¹ of the biscarbonate compound (III) in the present invention.

The biscarbonate compound (III) can be produced by reacting the divalent alcohol compound (II) and diphenyl carbonate, which is developed as the replacement of phosgene, as a conventional method, but phenol is produced as a by-product from diphenyl carbonate in this method. For example, when phenol is tried to be removed from the biscarbonate compound (III) by distillation, phenol is difficult to completely remove, since phenol is a solid under ordinary temperature and thus the viscosity of the crude composition increases with progression of distillation. The inventors of the present invention have experimentally found that the remaining phenol inhibits a polymerization reaction and also remains in the target polyurethane. The inventors found that the remaining phenol causes coloration of polyurethane, since phenol is very susceptible to oxidation.

The fluorocarbonate compound (I) is used in the present invention against the prior art. When the fluorocarbonate compound (I) and the divalent alcohol compound (II) are reacted, a fluoroalcohol is produced as a by-product but can be distilled more easily than phenol. Even if a fluoroalcohol remains in polyurethane, a fluoroalcohol is much more difficult to be oxidized than phenol. Thus, a fluoroalcohol may not adversely affect the transparency of polyurethane and may give preferred properties, such as repellency, antifouling property, weather resistance and abrasion resistance, to polyurethane on the basis of a fluoro group.

The Rf in the fluorocarbonate compound (I) is independently an aliphatic hydrocarbon group having a fluoro group. An example of the aliphatic hydrocarbon group having a fluoro group includes a C₁₋₁₀ monovalent chain aliphatic hydrocarbon group having a fluoro group, a C₁₋₁₀ monovalent cyclic aliphatic hydrocarbon group having a fluoro group, and a monovalent organic group formed by binding 2 or more and 5 or less of the above groups.

The "C₁₋₁₀ monovalent chain aliphatic hydrocarbon group" means a linear or branched monovalent saturated or unsaturated aliphatic hydrocarbon group having a carbon number of 1 or more and 10 or less. An example of the C₁₋₁₀ monovalent chain aliphatic hydrocarbon group includes a C₁₋₁₀ alkyl group, a C₂₋₁₀ alkenyl group and a C₂₋₁₀ alkynyl group.

An example of the C₁₋₁₀ alkyl group includes methyl, ethyl, n-propyl, isopropyl, n-butyl, 1-methylpropyl, 2-methylpropyl, 1,1-dimethylethyl, 2,2-dimethylethyl, n-pentyl, n-hexyl, 2-hexyl, 3-hexyl, 4-methyl-2-pentyl, n-heptyl, n-octyl and n-decyl. The group is preferably a C₂₋₈ alkyl group and more preferably a C₄₋₆ alkyl group.

An example of the C₂₋₁₀ alkenyl group includes ethenyl (vinyl), 1-propenyl, 2-propenyl (allyl), butenyl, hexenyl, octenyl and decenyl. The group is preferably a C₂₋₈ alkenyl group and more preferably a C₄₋₆ alkenyl group.

An example of the C₂₋₁₀ alkynyl group includes ethynyl, propynyl, butynyl, hexynyl, octynyl and pentadecynyl. The group is preferably a C₂₋₈ alkynyl group and more preferably a C₂₋₆ alkynyl group.

The "C₃₋₁₀ monovalent cyclic aliphatic hydrocarbon group" means a cyclic saturated or unsaturated aliphatic hydrocarbon group having a carbon number of 1 or more and 10 or less. An example of the group includes a C₃₋₁₀ cycloalkyl group, a C₄₋₁₀ cycloalkenyl group and a C₄₋₁₀ cycloalkynyl group.

An example of the monovalent organic group formed by binding 2 or more and 5 or less of the C₁₋₁₀ monovalent chain aliphatic hydrocarbon group and the C₃₋₁₀ monovalent cyclic aliphatic hydrocarbon group includes a C₃₋₁₀ monovalent cyclic aliphatic hydrocarbon group - C₁₋₁₀ divalent chain aliphatic hydrocarbon group and a C₁₋₁₀ monovalent chain aliphatic hydrocarbon group - C₃₋₁₀ divalent cyclic aliphatic hydrocarbon group - C₁₋₁₀ divalent chain aliphatic hydrocarbon group.

The substituent number of the fluoro group on the aliphatic hydrocarbon group having a fluoro group is not particularly restricted as long as the group can be substituted, and is preferably 2 or more and more preferably 3 or more, since the reactivity of the fluorocarbonate compound (I) becomes higher due to larger number of the fluoro group. With respect to the upper limit of the substituent number, the substituent number may be adjusted to, for example, 20 or less and preferably 15 or less. The aliphatic hydrocarbon group is preferably a sec-alkyl group or a tert-alkyl group and more preferably a perfluoro sec-alkyl group or tert-alkyl group, of which all hydrogen atoms are substituted with fluoro groups at the carbon atoms other than the carbon at the 1^{st} position. The fluorocarbonate compound (I) may be further substituted with a halogeno group selected from chloro group, bromo group and iodo group as an equally electron-withdrawing group in addition to fluoro group.

If the fluorocarbonate compound (I) is commercially available, the product may be used. Alternatively, the fluorocarbonate compound (I) may be synthesized. For example, the fluorocarbonate compound (I) can be synthesized by an ordinary method using phosgene, by the reaction of a fluoro aliphatic hydrocarbon ester of trichloroacetic acid and a fluoroalcohol, or by the method described in WO 2018/211953 without using phosgene.

An example of the fluorocarbonate compound (I) includes bis(2,2,2-trifluoroethyl)carbonate, bis(2,2,3,3-tetrafluoropropyl)carbonate, bis(1,1,1,3,3,3-hexafluoroisopropyl)carbonate, bis(1,1,1,2,2,4,5,5,5-nonafluoro-4-trifluoromethyl-3-pentyl)carbonate, bis[1,1,1,3,3,3-hexafluoro-2-(trifluoromethyl)propane-2-yl]carbonate, bis(2,2,3,3,3-pentafluoropropyl)carbonate, bis(2,2,3,3,4,4,5,5-octafluoropentyl)carbonate and bis(2,2,3,3,4,4,5,5-octafluorocyclopentyl)carbonate.

The R¹ in the divalent alcohol compound (II) is a divalent organic group. An example of the R¹ includes a C₁₋₁₀ divalent chain aliphatic hydrocarbon group, a C₃₋₁₀ divalent cyclic aliphatic hydrocarbon group, a C₆₋₁₅ divalent aromatic hydrocarbon group, and a divalent organic group formed by bonding 2 or more and 5 or less of the groups.

The "C₁₋₁₀ divalent chain aliphatic hydrocarbon group" means a linear or branched divalent saturated or unsaturated aliphatic hydrocarbon group having a carbon number of 1 or more and 10 or less. An example of the C₁₋₁₀ divalent chain aliphatic hydrocarbon group includes a C₁₋₁₀ alkanediyl group, a C₂₋₁₀ alkenediyl group and a C₂₋₁₀ alkynediyl group.

An example of the C₁₋₁₀ alkanediyl group includes methylene, ethylene, n-propylene, isopropylene, n-butylene, 1-methylpropylene, 2-methylpropylene, 1,1-dimethylethylene, 2,2-dimethylethylene, n-pentylene, n-hexylene, n-heptylene, n-octylene and n-decylene. The group is preferably a C₂₋₁₀ alkanediyl group or a C₁₋₈ alkanediyl group and more preferably a C₁₋₆ alkanediyl group or a C₁₋₄ alkanediyl group.

An example of the C₂₋₁₀ alkenediyl group includes ethenylene (vinylene), 1-propenylene, 2-propenylene (allylene), butenylene, hexenylene, octenylene and decenylene. The group is preferably a C₂₋₈ alkenediyl group and more preferably a C₂₋₆ alkenediyl group or a C₂₋₄ alkenediyl group.

An example of the C₂₋₁₀ alkynediyl group includes ethynylene, propynylene, butynylene, hexynylene, octynylene and pentadecynylene. The group is preferably a C₂₋₈ alkynediyl group and more preferably a C₂₋₆ alkynediyl group or a C₂₋₄ alkynediyl group.

The "C₃₋₁₀ divalent cyclic aliphatic hydrocarbon group" means a cyclic divalent saturated or unsaturated aliphatic hydrocarbon group having a carbon number of 1 or more and 10 or less. An example of the group includes a C₃₋₁₀ cycloalkanediyl group, a C₃₋₁₀ alkenediyl group and a C₃₋₁₀ cycloalkynediyl group. An example of the C₃₋₁₀ cycloalkanediyl group includes cyclobutanediyl, cyclopropanediyl, cyclohexanediyl and adamantanediyl.

The "C₆₋₁₅ divalent aromatic hydrocarbon group" means a divalent aromatic hydrocarbon group having a carbon number of 6 or more and 15 or less. An example of the group includes phenylene, indenylene, naphthylene, biphenylene, phenalenylene, phenanthrenylene and anthracenylene. The group is preferably a C₆₋₁₂ divalent aromatic hydrocarbon group and more preferably phenylene.

An example of the divalent organic group formed by binding 2 or more and 5 or less of the groups selected from the C₁₋₁₀ divalent chain aliphatic hydrocarbon group, the C₃₋₁₀ divalent cyclic aliphatic hydrocarbon group and the C₆₋₁₅ divalent aromatic hydrocarbon group includes a C₃₋₁₀ divalent cyclic aliphatic hydrocarbon group - C₁₋₁₀ divalent chain aliphatic hydrocarbon group, a C₁₋₁₀ divalent chain aliphatic hydrocarbon group - C₃₋₁₀ divalent cyclic aliphatic hydrocarbon group, a C₆₋₁₅ divalent aromatic hydrocarbon group - C₁₋₁₀ divalent chain aliphatic hydrocarbon group, a C₁₋₁₀ divalent chain aliphatic hydrocarbon group - C₆₋₁₅ divalent aromatic hydrocarbon group, a C₁₋₁₀ divalent chain aliphatic hydrocarbon group - C₃₋₁₀ divalent cyclic aliphatic hydrocarbon group - C₁₋₁₀ divalent chain aliphatic hydrocarbon group, a C₃₋₁₀ divalent cyclic aliphatic hydrocarbon group - C₁₋₁₀ divalent chain aliphatic hydrocarbon group - C₃₋₁₀ divalent cyclic aliphatic hydrocarbon group and a C₁₋₁₀ divalent chain aliphatic hydrocarbon group - C₆₋₁₅ divalent aromatic hydrocarbon group - C₁₋₁₀ divalent chain aliphatic hydrocarbon group.

The above-described divalent organic group in the divalent alcohol compound (II) may be substituted with 1 or more halogeno groups selected from fluoro, chloro, bromo and iodo. The C₃₋₁₀ divalent cyclic aliphatic hydrocarbon group and the C₆₋₁₅ divalent aromatic hydrocarbon group may have an ether group (-O-) and may be further substituted with a C₁₋₆ alkyl group in addition to a halogeno group. The substituent group is preferably fluoro.

An example of the divalent alcohol compound (II) includes ethanediol, propanediol, butanediol, pentanediol, hexanediol, heptanediol, octanediol and isosorbide.

An example of the R¹ in the divalent alcohol compound includes a divalent organic group represented by the following formula (VI): 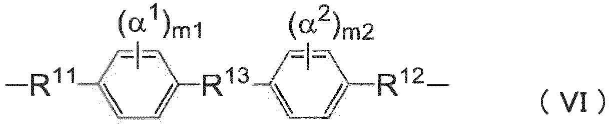 wherein
R¹¹ and R¹² are independently -(CR¹⁴R¹⁵)ₘ₃- or -(-O-(CR¹⁴R¹⁵)ₘ₄-)ₘ₅- (wherein R¹⁴ and R¹⁵ are independently H or a C₁₋₆ alkyl group, m3 is an integer of 0 or more and 10 or less, m4 is an integer of 1 or more and 10 or less, m5 is an integer of 1 or more and 10 or less, and when m3 or m4 is an integer 2 or more, a plurality of R¹⁴ and R¹⁵ may be the same as or different from each other),
R¹³ is any one of the following divalent organic groups: 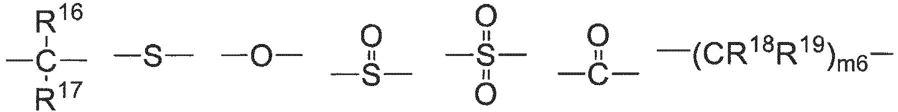 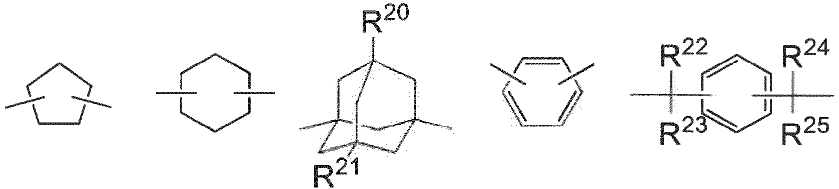 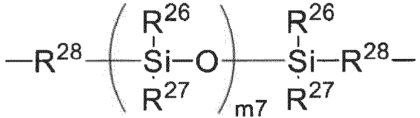 (wherein
   R¹⁶ and R¹⁷ are independently H, a halogeno group, a C₁₋₂₀ aliphatic hydrocarbon group optionally having a substituent β, a C₁₋₂₀ alkoxy group optionally having a substituent β, a C₆₋₂₀ aromatic hydrocarbon group optionally having a substituent γ, or R¹⁶ and R¹⁷ may be bound to form a C₃₋₂₀ carbon ring or a 5-12 membered heterocyclic ring,
   R¹⁸ and R¹⁹ are independently H or a C₁₋₆ alkyl group, and when m6 is an integer of 2 or more, a plurality of R¹⁸ and R¹⁹ may be the same as or different from each other,
   R²⁰ to R²⁷ are independently a halogeno group, a C₁₋₂₀ aliphatic hydrocarbon group optionally having a substituent β, a C₁₋₂₀ alkoxy group optionally having a substituent β, or a C₆₋₁₂ aromatic hydrocarbon group optionally having a substituent γ,
   R²⁸ is a C₁₋₉ alkanediyl group optionally having a substituent β,
   m6 is an integer of 1 or more and 20 or less,
   m7 is an integer of 1 or more and 500 or less.)
   a substituent α¹ and a substituent α² are independently a substituent group selected from the group consisting of a halogeno group, a C₁₋₂₀ aliphatic hydrocarbon group, a C₁₋₂₀ alkoxy group, a C₃₋₂₀ cycloalkyl group, a C₆₋₂₀ aromatic hydrocarbon group, a C₇₋₂₀ aralkyl group, a C₆₋₂₀ aromatic hydrocarbonoxy group and a C₃₋₂₀ cycloalkoxy group,
   m1 and m2 are independently integers of 0 or more and 4 or less,
   the substituent β is 1 or more substituent groups selected from a C₁₋₆ alkoxy group, a C₁₋₇ acyl group, a halogeno group, a nitro group, a cyano group and a carbamoyl group,
   the substituent γ is 1 or more substituent groups selected from a C₁₋₆ alkyl group, a C₁₋₆ alkoxy group, a C₁₋₇ acyl group, a halogeno group, a nitro group, a cyano group and a carbamoyl group.

An example of the -Ph-R¹³-Ph- in the divalent organic group (VI) includes a divalent organic group as the part except for the hydroxy groups in bisphenol A, bisphenol AP, bisphenol AF, bisphenol B, bisphenol BP, bisphenol C, bisphenol E, bisphenol F, bisphenol G, bisphenol S, bisphenol TMC and bisphenol Z.

An example of the -(CR¹⁴R¹⁵)ₘ₃- group in the divalent organic group (VI) includes a single bond and a C₁₋₂ alkyl group, and an example of the -(-O-(CR¹⁴R¹⁵)ₘ₄-)ₘ₅- group includes -(-O-CH₂CH₂-)ₘ₅-, -(-O-CH(CH₃)CH₂-)ₘ₅- and -(-O-CH₂CH(CH₃)-)ₘ₅-.

An example of the R¹ in the divalent alcohol compound (II) includes a divalent organic group represented by the formula - R³-[-X-R³-]ₘ- (wherein X is O or S and preferably O, R³ is a C₁₋₈ alkanediyl group, m is an integer of 1 or more and 180 or less, and when m is an integer of 2 or more, a plurality of X and R³ are the same as or different from each other.).

An example of the R³ includes ethylene group (-CH₂CH₂-), propylene group [-CH(CH₃)CH₂- or -CH₂CH(CH₃)-] and tetramethylene group (-CH₂CH₂CH₂CH₂-).

The m is preferably 5 or more, more preferably 10 or more, even more preferably 20 or more, and preferably 160 or less, more preferably 150 or less.

When the fluorocarbonate compound (I) and the divalent alcohol compound (II) are reacted, a solvent may be used. The solvent is not particularly restricted as long as the solvent is liquid under ordinary temperature and ordinary pressure and does not have a bad effect on the reaction. An example of the solvent includes a nitrile solvent such as acetonitrile; an ether solvent such as diethyl ether, glyme, diglyme, triglyme, tetraglyme, tetrahydrofuran and dioxane; a ketone solvent such as acetone and methyl ethyl ketone; an ester solvent such as ethyl acetate; and a halogenated hydrocarbon solvent such as dichloromethane, chloroform and carbon tetrachloride. When at least one of the fluorocarbonate compound (I) and the divalent alcohol compound (II) is liquid under the reaction condition, a solvent may not be used. It is preferred not to use a solvent in terms of a cost and an environmental load.

The amounts of the fluorocarbonate compound (I) and the divalent alcohol compound (II) may be appropriately adjusted. For example, when one of the fluorocarbonate compound (I) and the divalent alcohol compound (II) is difficult to synthesize or is expensive, a molar ratio of the other compound to the compound can be adjusted to five times or more by mole and 20 times or less by mole. The molar ratio is preferably 15 times or less by mole and more preferably 12 times or less by mole. In addition, the molar ratio may be adjusted to 0.5 times or more by mole and 1.5 times or less by mole.

The fluorocarbonate compound (I) and the divalent alcohol compound (II) may be reacted in the presence of a base in the Step 1. An example of the base includes an organic base such as pyridine, triethylamine, ethyldiisopropylamine, diazabicycloundecene (DBU) and N-methylmorpholine; and an inorganic base such as sodium hydrogencarbonate, potassium hydrogencarbonate, sodium carbonate, potassium carbonate, cesium carbonate and calcium carbonate. The base is preferably an organic base in terms of the solubility in the reaction liquid and the appropriate basicity. The use amount of the base may be appropriately adjusted. For example, 0.01 times or more by mole and 1 time or less by mole of the base to the smaller molar number of the fluorocarbonate compound (I) and the divalent alcohol compound (II) may be used. It is preferred not to use the base in terms of a cost and the residual base.

The reaction temperature may be appropriately adjusted and may be adjusted to, for example, 30°C or higher and 120°C or lower. The reaction can be carried out under a heating and refluxing condition depending on the solvent. The reaction time may be also appropriately adjusted. The reaction may be carried out until it is confirmed by chromatography or the like that at least one of the fluorocarbonate compound (I) and the divalent alcohol compound (II) is consumed. The reaction time may be determined by a preliminary experiment. The reaction time may be adjusted to, for example, 1 hour or more and 50 hour or less.

A general aftertreatment may be carried out after the reaction. For example, water and a water-insoluble solvent such as diethyl ether, chloroform and ethyl acetate are added to the reaction liquid, and the organic phase and the aqueous phase are separated. The biscarbonate compound (III) as the target compound is extracted into the organic phase. The organic phase may be washed using water and saturated sodium chloride aqueous solution, and may be dried over anhydrous sodium sulfate and anhydrous magnesium sulfate. The organic phase is concentrated to obtain the biscarbonate compound (III). The biscarbonate compound (III) may be further purified by chromatography, recrystallization or the like, or may be directly used in the following Step 2.

The reaction is accelerated in the case of the above-described biscarbonate compound (III-1) among the biscarbonate compound (III), since a fluoroalcohol is particularly easily eliminated by the reaction. In particular, when R¹ does not contain a fluoro group, the reaction may hardly progress in some cases; on the one hand, polyurethane can be successfully produced even in such a case by using the biscarbonate compound (III-1), since the biscarbonate compound (III-1) is particularly highly reactive.

### 2. Step to produce polyurethane

The biscarbonate compound (III) obtained in the above Step 1 and the divalent amino compound (IV) are reacted to obtain the polyurethane (V) in this step.

The R² in the divalent amino compound (IV) is a divalent organic group. An example of the R² in the divalent amino compound (IV) includes a divalent organic group exemplified as the R¹ in the divalent alcohol compound (II). The R² in the divalent amino compound (IV) may be the same as or different from the R¹ in the divalent alcohol compound (II). The R¹ and the R² are preferably different from each other in terms of various properties of the polyurethane (V) as the target compound.

When the biscarbonate compound (III) and the divalent amino compound (IV) are reacted, a solvent may be used. The solvent is not particularly restricted as long as the solvent is liquid under ordinary temperature and ordinary pressure and does not have a bad effect on the reaction. An example of the solvent includes an aromatic hydrocarbon such as benzene, toluene and chlorobenzene; a nitrile solvent such as acetonitrile; an ether solvent such as diethyl ether, tetrahydrofuran and dioxane; an ester solvent such as ethyl acetate; a halogenated hydrocarbon solvent such as dichloromethane, chloroform and carbon tetrachloride; a hydrocarbon solvent such as pentane and hexane; a ketone solvent such as acetone and methyl ethyl ketone; an amide solvent such as dimethylformamide and dimethylacetamide; and a sulfoxide solvent such as dimethylsulfoxide. When at least one of the biscarbonate compound (III) and the divalent amino compound (IV) is liquid under the reaction condition, a solvent may not be used. It is preferred not to use a solvent in terms of a cost and an environmental load.

The amounts of the biscarbonate compound (III) and the divalent amino compound (IV) may be appropriately adjusted. For example, a molar ratio of the divalent amino compound (IV) to 1 mole of the biscarbonate compound (III) may be adjusted to 0.5 times or more by mole and 1.5 times or less by mole. The molar ratio is preferably 0.8 times or more by mole, more preferably 0.9 times or more by mole, and preferably 1.2 times or less by mole, more preferably 1.1 times or less by mole.

The reaction temperature of this Step 2 may be appropriately adjusted and may be adjusted to, for example, 10°C or higher and 200°C or lower. The reaction can be carried out under a heating and refluxing condition depending on the solvent. The reaction time may be also appropriately adjusted. The reaction may be carried out until it is confirmed by chromatography or the like that at least one of the biscarbonate compound (III) and the divalent amino compound (IV) is consumed. The reaction time may be determined by a preliminary experiment. The reaction time may be adjusted to, for example, 30 minutes or more and 10 hours or less.

A general aftertreatment may be carried out after the reaction. For example, the polyurethane (V) as the target compound may be washed with an inactive solvent such as n-hexane, since the polyurethane (V) is a polymer and is insoluble in a solvent. The polyurethane (V) may be dried after washing or without washing.

Polyurethane can be produced easily, safely and efficiently without using a highly toxic isocyanate compound by the present invention. The polyurethane produced by the present invention can have desired properties such as high strength, flexibility and water repellency due to the two divalent organic groups in the molecular structure unit, i.e. the combination of the R¹ in the biscarbonate compound (III) and the R² in the divalent amino compound (IV). In addition, the quality of the polyurethane may be improved due to the remaining fluoroalcohol.

The present application claims the benefit of the priority date of Japanese patent application No. 2020-83148 filed on May 11, 2020.

### EXAMPLES

Hereinafter, the examples are described to demonstrate the present invention more specifically, but the present invention is in no way restricted by the examples, and the examples can be appropriately modified to be carried out within a range that adapts to the contents of this specification. Such a modified example is also included in the range of the present invention.

### Example 1: Synthesis of hardly yellowing thermoplastic polyurethane

### (1) Synthesis of 1,6-hexamethylene bis(1,1,1,2,2,4,5,5,5-nonafluoro-4-(trifluoromethyl)-3-pentyl carbonate

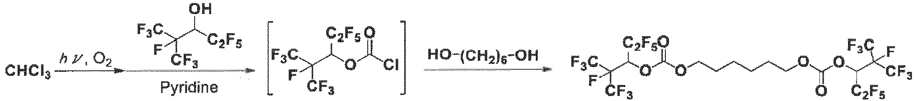

Chloroform (50 mL, 620 mmol) was added into a three-neck cylindrical flask of φ 42 mm × 200 mm, and light was irradiated thereto using a low pressure mercury lamp of 20 W and Φ 24 mm × 120 mm at 0°C for 3 hours with blowing oxygen bubble at a flow rate of 1.0 L/min. The lamp was placed at 2 cm from the bottom of the flask. The light contained ultraviolet of 253.7 nm and 184.9 nm. Then, the light was turned off, the reaction liquid was cooled to -30°C, and 1,1,1,2,2,4,5,5,5,-nonafluoro-4-(trifluoromethyl)pentanol (3.2 g, 10 mmol) and pyridine (3.2 mL, 30 mmol) were successively added thereto. The mixture was stirred for 2 hours. Next, the reaction mixture was warmed to 50°C and stirred for 2 hours to remove a photolysis gas such as phosgene dissolved in the reaction mixture from the reaction mixture. The generated gas was passed through a saturated sodium hydrogencarbonate aqueous solution to be degraded to carbon dioxide gas and to be discharged. Then, 1,6-hexanediol (0.35 g, 3 mmol) was added, and the mixture was stirred at 30°C for 15 hours. Dichloromethane and water were added to the reaction mixture, and the organic phase and the aqueous phase were separated. The organic phase was dried over anhydrous sodium sulfate. The solvent was evaporated under reduced pressure, and the target compound as colorless liquid was obtained by distillation using a glass tube oven (yield: 60%, amount: 1.4 g, 1.8 mmol). ¹H NMR (400 MHz, CDCl₃, 20°C): δ/ppm = 6.01(ttt, J=19Hz, 6.6Hz, 3.6Hz, 2H, methine), 4.29(t, J=6.6Hz, 4H, methylene), 1.72(t, J=6.6Hz, 4H, methylene), 1.40(tt, J=6.6Hz, 4H, methylene) ¹⁹F NMR (376 MHz, CDCl₃, 20°C): δ ppm = -73.87 (m, 6F, -CF₃), - 83.06(m, 3F, -CF₃), -122.90(m, 2F, -CF₂-), -182.84(m, 1F, -CF-) ¹³C NMR (125 MHz, CDCl₃, 20°C): δ ppm = 152.36, 119.92, 119.50, 119.261, 110.79, 89.65, 70.64, 67.84, 28.28, 25.11 IR (ATR): 2972, 2871, 1780, 1302, 1206, 1171, 1115, 970 cm⁻¹ FAB-MS: m/z calculated for [M+H]⁺ (C₂₀H₁₄F₂₄O₆) 807.04, found 806.91

### (2) Synthesis of hardly yellowing thermoplastic polyurethane

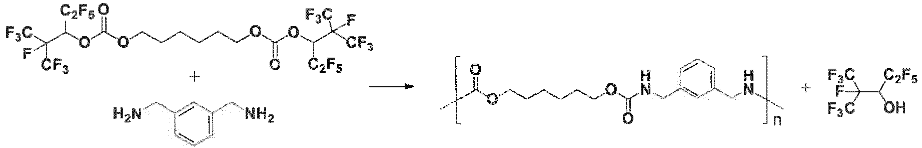

Into a 10 mL sample bottle, 1,6-hexamethylene bis(1,1,1,2,2,4,5,5,5-nonafluoro-4-(trifluoromethyl)-3-pentyl carbonate) (0.20 g, 0.5 mmol), m-xylylenediamine (0.07 g, 0.5 mmol) and toluene (3 mL) as a solvent were added. The mixture was stirred at 100°C for 1 hour. The reaction mixture was washed with n-hexane and was subjected to suction filtration. The residue was dried in vacuo at 60°C for 2 hours to obtain the target compound as pale yellow solid (yield: 61%, amount: 93 mg, 0.3 mmol).
¹H NMR (400 MHz, DMSO-d₆, 20°C): δ7.64(br., 2H, NH), 7.35-7.09(br., 4H, Phenyl), 4.14(d, 4H, methylene), 3.94(t, 4H, methylene), 1.53(br., 4H, methylene), 1.31(br., 4H, methylene) IR (ATR): 3304, 2931, 1686, 1523, 1240, 1135, 1048 cm⁻¹ Average molecular weight by HPLC (polystyrene standard): Mₙ = 3100, M_{w} = 5200, M_{w}/Mₙ = 1.7

### Example 2: Synthesis of hardly yellowing thermoplastic polyurethane

### (1) Synthesis of PPG bis(2,2,2-trifluoroethyl carbonate) [B3FEC + polypropylene glycol 400 (PPG)]

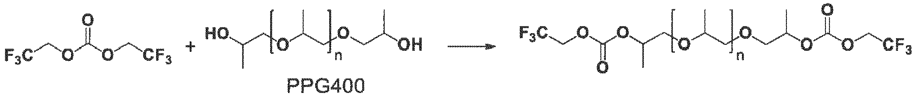

Bis(2,2,2-trifluoroethyl carbonate) (3.39 g, 15.0 mmol) and potassium carbonate (55 mg, 0.4 mmol) were added to acetonitrile (2 mL). The mixture was mixed to prepare a solution. Polypropylene glycol 400 (2.0 g, 5.0 mmol) was added to the solution, and the mixture was stirred at 50°C for 3 hours. Dichloromethane and water were added to the reaction mixture, and the organic phase and the aqueous phase were separated. The organic phase was dried over anhydrous sodium sulfate. The solvent was evaporated under reduced pressure to obtain the target compound as pale yellow oil liquid (yield: 90%, amount: 2.92 g, 4.47 mmol).
¹H NMR (400 MHz, CDCl₃, 293 K): δ/ppm = 4.94(sext, J=6.0Hz, 2H, methine), 4.50(q, J=8.3Hz, 4H, methylene), 3.39-3.62(m, methylene, methine), 1.31(dd, J=6.8Hz, 3.2Hz, 6H, methyl), 1.13(m, methyl)
¹⁹F NMR (376 MHz, CDCl₃, 293 K): δ/ppm = -74.20
IR (ATR): ν = 2974, 2876, 1759, 1296, 1246, 1166, 1102, 991, 956 cm⁻¹
ESI-FT-MS: m/z calculated for [M+Na]⁺ [C₁₂H₁₆F₆O₇(C₃H₆O)ₙ] 409.07 + n (58.04) (n≧3), found 583.20 (n=3), 641.24 (n=4), 699.28 (n=5), 757.32 (n=6), 815.37 (n=7), 873.41 (n=8), 931.45 (n=9)

### (2) Synthesis of hardly yellowing thermoplastic polyurethane

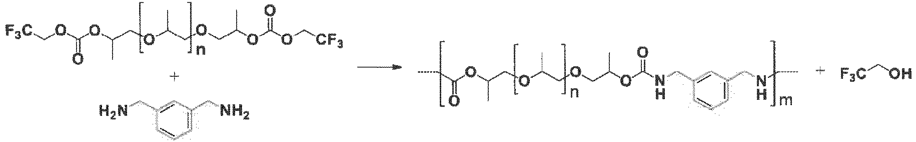

Polypropylene glycol bis(2,2,2-trifluoroethyl carbonate) (1.96 g, 3.0 mmol) and m-xylylenediamine (0.41 g, 3.0 mmol) were added into a 20 mL one-neck round-bottom flask, and the mixture was stirred at 150°C for 3 hours. The reaction mixture was dried in vacuo at 50°C for 3.5 hours to obtain the target compound as brown oil (yield: >99%, amount: 1.8 g, 3.0 mmol). ¹H NMR (400 MHz, CDCl₃, 20°C): δ7.28-7.30(br., 1H, Phenyl), 7.17-7.19(br., 3H, Phenyl), 5.31(br., 2H, NH), 4.93(br., 2H, methine), 4.32(br., 4H, methylene), 3.38-3.54[m, methine and methylene], 1.23(br., 6H, methyl), 1.12(br., methyl)
IR (ATR): 3316, 2974, 2929, 2873, 1697, 1531, 1450, 1376, 1247, 1098, 944 cm⁻¹
Average molecular weight by HPLC (polystyrene standard): Mₙ = 2900, M_{w} = 4700, M_{w}/Mₙ = 1.6

### Example 3: Synthesis of hardly yellowing thermoplastic polyurethane

### (1) Synthesis of 1,6-hexamethylene bis(1,1,1,3,3,3-hexafluoroisopropyl carbonate)

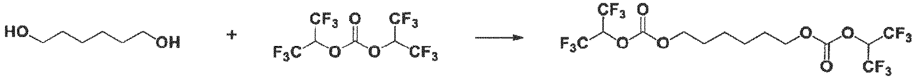

Bis(1,1,1,3,3,3-hexafluoroisopropyl carbonate) (BHFC) (3.62 g, 10 mmol), 1,6-hexanediol (0.12 g, 1.0 mmol) and triethylamine (0.1 mmol, 13.8 µL) were added into a one-neck round-bottom flask, and the mixture was stirred at 90°C for 3 hours. The reaction mixture was dried in vacuo at 50°C for 3 hours to obtain the target compound as colorless oil (yield: 98%, amount: 0.50 g, 0.98 mmol).
¹H NMR (400 MHz, CDCl₃, 293 K): δ/ppm = 5.56(m, 2H, methine), 4.30(t, J=6.8Hz, 3H, methylene), 1.79-1.72(m, 4H, methylene), 1.47-1.43(m, 4H, methylene)
¹³C NMR (125 MHz, DMSO-d₆, 293 K): δ/ppm = 153.2, 120.5, 70.3, 70.1, 28.4, 25.3
¹⁹F NMR (376 MHz, CDCl₃, 293 K): δ/ppm = -73.57
IR (ATR): 1771, 1385, 1364, 1300, 1248, 1230, 1194, 1141, 1107, 915, 906, 688, 601, 563 cm⁻¹
FAB-MS: m/z calculated for [M+H]⁺ (C₁₄H₁₄F₁₂O₆) 507.07, found 506.96

### (2) Synthesis of hardly yellowing thermoplastic polyurethane

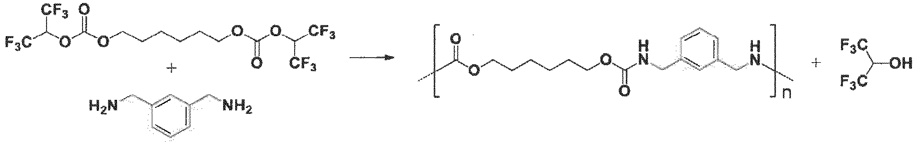

Into a 50 mL round-bottom flask, 1,6-hexamethylene bis(1,1,1,3,3,3-hexafluoroisopropyl carbonate) (0.4 g, 0.8 mmol) and m-xylylenediamine (0.1 g, 0.8 mmol) were added. The mixture was stirred at 150°C for 1.5 hours. The reaction mixture was dried in vacuo at 50°C for 2 hours to obtain the target compound as white solid (yield: >99%, amount: 0.27 g, 0.9 mmol).
¹H NMR (400 MHz, DMSO-d₆, 293 K): δ/ppm = 7.63(t, J=5.2Hz, 2H, NH), 7.24(t, J=8.0Hz, 1H, phenyl), 7.10-7.09(br., 3H, phenyl), 4.14(d, J=5.6Hz, 4H, benzyl), 3.94(t, J=6.4Hz, 4H, methylene), 1.54(br., 4H, methylene), 1.32(br., 4H, methylene)
IR (ATR): 3306, 2934, 1684, 1526, 1476, 1249, 1132, 1072, 1049 cm⁻¹
Average molecular weight by HPLC (polystyrene standard): unmeasurable due to a large amount of insoluble solid

### Example 4: Synthesis of hardly yellowing thermoplastic polyurethane

### (1) Synthesis of polypropylene glycol bis(1,1,1,3,3,3-hexafluoroisopropyl carbonate)

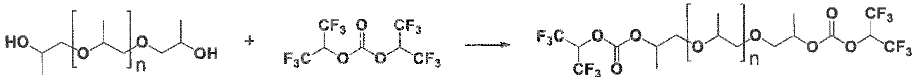

Bis(1,1,1,3,3,3-hexafluoroisopropyl carbonate) (BHFC) (3.62 g, 10 mmol), polypropylene glycol 400 (0.4 g, 1.0 mmol) and triethylamine (0.1 mmol, 13.8 µL) were added into a two-neck round-bottom flask, and the mixture was stirred at 90°C for 21 hours. The reaction mixture was dried in vacuo at 50°C for 3 hours to obtain the target compound as colorless oil (yield: 99%, amount: 0.78 g, 0.99 mmol).
¹H NMR (400 MHz, CDCl₃, 293 K): δ/ppm = 5.57(m, 2H, methine), 5.05-4.97(m, 2H, methine), 3.65-3.38(m, 19H, methylene + methine), 1.35-1.32(m, 6H, methyl), 1.14-1.10(m., 15H, methyl) ¹⁹F NMR (376 MHz, CDCl₃, 293 K): δ/ppm = -73.53
IR (ATR): 2980, 2900, 2875, 2866, 1773, 1385, 1364, 1299, 1256, 1227, 1198, 1168, 1109, 1078, 1068, 1008, 921, 908, 894, 689 cm⁻¹
ESI-FT-MS: m/z calculated for [M+Na]⁺ 545.04 + n (58.04), found 777.21 (n=4), 835.25 (n=5), 893.30 (n=6), 951.34 (n=7), 1009.38 (n=8), 1067.42 (n=9), 1125.46 (n=10), 1183.50 (n=11)

### (2) Measurement of remaining chlorine

The obtained biscarbonate (50 mg) was burned with P as an internal standard and absorbed in an absorbing liquid using Automatic Quick Furnace ("AQF-2100" manufactured by Mitsubishi Analytech). As the absorbing liquid, 25 mM NaOH + 0.1% H₂O₂ was used. The obtained absorbing liquid was analyzed by ion chromatography ("ICS-2100" manufactured by Thermo Fisher Scientific, column: AS11HC) to determine the amounts of Cl and P in the absorbing liquid. As a result, the chlorine concentration was 132 wtppm. The mixed chlorine may be derived from the impurity contained in BHFC used as a raw material.

### (3) Synthesis of hardly yellowing thermoplastic polyurethane

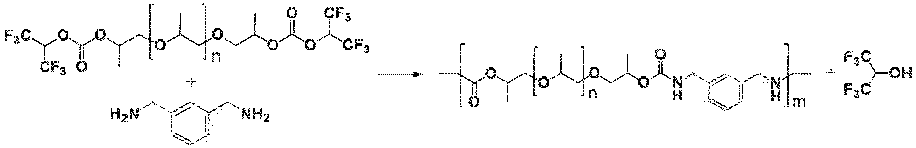

Polypropylene glycol bis(1,1,1,3,3,3-hexafluoroisopropyl carbonate) (2.2 g, 3.0 mmol) and m-xylylenediamine (0.4 g, 3.0 mmol) were added into a 50 mL round-bottom flask, and the mixture was stirred under argon atmosphere at 150°C for 3 hours. The reaction mixture was dried in vacuo at 50°C for 2 hours to obtain the target compound as pale yellow oil (yield: 90%, amount: 1.57 g, 2.7 mmol).
¹H NMR (400 MHz, CDCl₃, 293 K): δ/ppm = 7.30(t, J=7.6Hz, 1H, phenyl), 7.19-7.17(br., 3H, phenyl), 5.30(br., 2H, NH), 4.94(br., 2H, methine), 4.32-4.25(br., 4H, benzyl), 3.64-3.28(br., 22H, methylene + methine), 1.25(d, 6H, methyl), 1.12-1.11(br., 18H, methyl)
IR (ATR): 3325, 2972, 2931, 1699, 1529, 1451, 1374, 1247, 1093, 753, 663, 553 cm⁻¹
Average molecular weight by HPLC (polystyrene standard): Mₙ = 27300, M_{w} = 46000, M_{w}/Mₙ = 1.68

The amount of the remaining chlorine in the obtained polyurethane was measured by the same method as the above-described (2). As a result, the amount of chlorine was very small as 30 wtppm. A fluoroalcohol is produced as the progress of the reaction but was not detected from the obtained polyurethane, since a fluoroalcohol is easily volatilized.

### Comparative example 1: Synthesis of polyurethane using diphenyl carbonate

### (1) Synthesis of polypropylene glycol bis(phenyl carbonate)

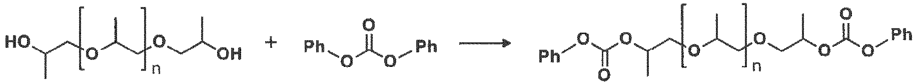

Polypropylene glycol 400 (0.4 g, 1.0 mmol), diphenyl carbonate (0.5 g, 2.5 mmol) and 1,4-diazabicyclo(2,2,2)octane (hereinafter described as DABCO) (0.01 g, 0.1 mmol) were added into a one-neck round-bottom flask, and the mixture was stirred at 100°C for 13 hours. The obtained oily reaction mixture was dried in vacuo at 100°C for 2 hours using an oil rotary pump to evaporate the eliminated alcohol produced by the reaction and DABCO to obtain a transparent oil product.

Since a small amount of the eliminated phenol remained in accordance with ¹H NMR spectrum, the product was further dried in vacuo at 200°C for 2 hours using an oil rotary pump to obtain a transparent oil product (yield: 67%).

### (2) Synthesis of polyurethane

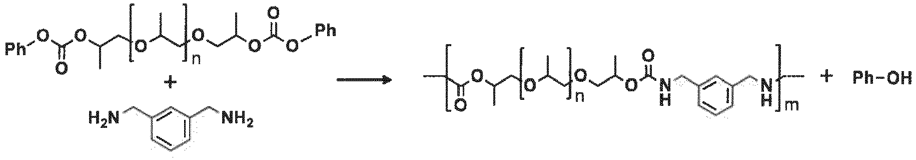

The bis(phenyl carbonate) (1.92 g, 3.0 mmol) obtained by Comparative example 1(1) and m-xylylenediamine (0.41 g, 3.0 mmol) were added into a 50 mL one-neck round-bottom flask, and the mixture was stirred at 150°C for 3 hours. The reaction mixture was dried in vacuo at 120°C for 4 hours using an oil rotary pump to remove the eliminated alcohol. It was confirmed that the corresponding polyurethane was produced by ¹H NMR spectrum.

The average molecular weight was determined by GPC; as a result, Mₙ was 3263, M_{w} was 6912 and M_{w}/Mₙ was 2.12. In the obtained polyurethane, 39% of phenol remained to the total phenol eliminated by the reaction.

As the above-described results, phenol remained in polyurethane, though the polyurethane was dried in vacuo under high temperature. The reason why the polyurethane has smaller molecular weight and larger variation of molecular weight than the polyurethane of Example 4(2) may be that phenol as a by-product could not be removed and inhibited the polymerization reaction.

### Example 5: Synthesis of non-yellowing thermoplastic polyurethane

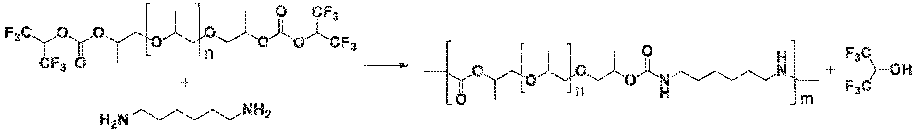

Polypropylene glycol bis(1,1,1,3,3,3-hexafluoroisopropyl carbonate) (2.3 g, 3.0 mmol) and 1,6-hexamethylenediamine (0.35 g, 3.0 mmol) were added into a 50 mL round-bottom flask, and the mixture was stirred under argon atmosphere at 150°C for 3 hours. The reaction mixture was dried in vacuo at 50°C for 2 hours to obtain the target compound as pale yellow oil (yield: >99%, amount: 1.81 g, 3.2 mmol). A fluoroalcohol is produced as the progress of the reaction but was not detected from the obtained polyurethane, since a fluoroalcohol is easily volatilized.
¹H NMR (400 MHz, CDCl₃, 293 K): δ/ppm = 4.93-4.87(br., 2H, methine), 3.65-3.30(br., 22H, methylene + methine), 3.14-3.13(br., 4H, methylene), 1.50-1.47(br., 4H, methylene), 1.34-1.30(br., 4H, methylene), 1.23(d, J=6.4Hz, 6H, methyl), 1.14(d, J=5.2Hz, 18H, methyl)
IR (ATR): 3329, 2972, 2931, 2862, 1697, 1530, 1453, 1374, 1252, 1200, 1097, 1016 cm⁻¹
Average molecular weight by HPLC (polystyrene standard): Mₙ = 10800, M_{w} = 17500, M_{w}/Mₙ = 1.62

### Comparative example 2: Synthesis of polyurethane using diphenyl carbonate

The bis(phenyl carbonate) (1.92 g, 3.0 mmol) obtained in Comparative example 1(1) and 1,6-hexamethylenediamine (0.35 g, 3.0 mmol) were added into a 50 mL one-neck round-bottom flask, and the mixture was stirred at 150°C for 3 hours. The reaction mixture was dried in vacuo using an oil rotary pump at 120°C for 4 hours to remove the eliminated alcohol. It was confirmed by ¹H NMR spectrum that the corresponding polyurethane was produced.

The average molecular weight was determined by GPC; as a result, Mₙ was 2791, M_{w} was 5804 and M_{w}/Mₙ was 2.08. In the obtained polyurethane, 62% of phenol remained to the total phenol eliminated by the reaction.

As the above-described results, phenol remained in polyurethane, though the polyurethane was dried in vacuo under high temperature. The reason why the polyurethane has smaller molecular weight and larger variation of molecular weight than the polyurethane of Example 5 may be that phenol as a by-product could not be removed and inhibited the polymerization reaction.

### Example 6: Synthesis of non-yellowing thermoplastic polyurethane

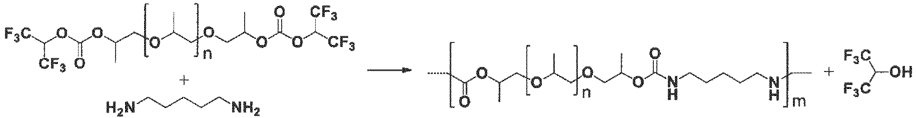

Polypropylene glycol bis(1,1,1,3,3,3-hexafluoroisopropyl carbonate) (0.39 g, 0.5 mmol) and 1,5-pentamethylenediamine (0.05 g, 0.5 mmol) were added into a 50 mL round-bottom flask, and the mixture was stirred at 100°C for 1 hour. The reaction mixture was dried in vacuo at 50°C for 2 hours to obtain the target compound as pale yellow oil (yield: 98%, amount: 0.27 g, 0.49 mmol).

¹H NMR (400 MHz, CDCl₃, 293 K): δ/ppm = 4.92-4.87(br., 2H, methine), 3.65-3.32(br., 16H, methylene + methine), 3.14-3.13(m., 4H, methylene), 1.51(m, 4H, methylene), 1.37-1.29(m, 2H, methylene), 1.22(br., 6H, methyl), 1.14-1.13(br., 12H, methyl)

IR (ATR): 3321, 2972, 2934, 2871, 1696, 1527, 1451, 1375, 1248, 1180, 1099, 1016 cm⁻¹

Average molecular weight by HPLC (polystyrene standard): Mₙ = 14300, M_{w} = 31000, M_{w}/Mₙ = 2.18

### Example 7: Synthesis of non-yellowing thermoplastic polyurethane

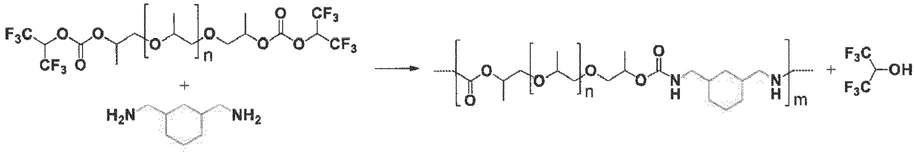

Polypropylene glycol bis(1,1,1,3,3,3-hexafluoroisopropyl carbonate) (0.39 g, 0.5 mmol) and 1,3-bis(aminomethyl)cyclohexane (0.07 g, 0.5 mmol) were added into a 50 mL round-bottom flask, and the mixture was stirred at 100°C for 2 hours. The reaction mixture was died in vacuo at 50°C for 2 hours to obtain the target compound as pale yellow oil (yield: 96%, amount: 0.29 g, 0.48 mmol).
¹H NMR (400 MHz, CDCl₃, 293 K): δ/ppm = 4.95-4.82 (br., 2H, methine), 3.59-3.39(br., 22H, methylene + methine), 3.07-2.95(br., 4H, methylene), 1.80-1.38 + 0.90-0.54(br., 10H, methylene + methine), 1.25-1.22(d, 6H, J=6.4Hz, methyl), 1.14-1.13(br., 18H, methyl)
IR (ATR): 3348, 2973, 2918, 2854, 1698, 1530, 1450, 1375, 1248, 1094, 1015 cm⁻¹
Average molecular weight by HPLC (polystyrene standard): Mₙ = 7400, M_{w} = 13200, M_{w}/Mₙ = 1.78

### Example 8: Synthesis of non-yellowing thermoplastic polyurethane

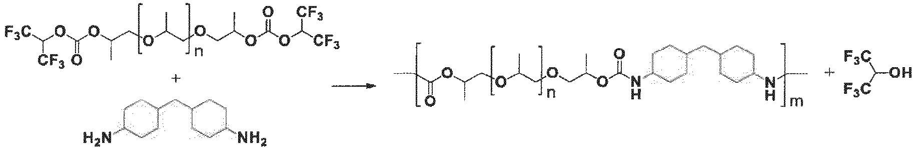

Polypropylene glycol bis(1,1,1,3,3,3-hexafluoroisopropyl carbonate) (0.79 g, 1.0 mmol) and 4,4'-methylene bis(cyclohexylamine) (0.21 g, 1.0 mmol) were added into a 50 mL round-bottom flask, and the mixture was stirred at 150°C for 1 hour. The reaction mixture was dried in vacuo at 50°C for 2 hours to obtain the target compound as pale yellow oil (yield: 98%, amount: 0.65 g, 0.98 mmol).
¹H NMR (400 MHz, CDCl₃, 293 K): δ/ppm = 4.95-4.82(br., 2H, methine), 3.59-3.39(br., 16H, methylene), 3.07-2.95(br., 4H, methylene), 1.25-1.22(br., 6H, methyl), 1.14-1.13(br., 12H, methyl)
IR (ATR): 3326, 2971, 2927, 2857, 1688, 1523, 1449, 1374, 1304, 1249, 1095, 1034 cm⁻¹
Average molecular weight by HPLC (polystyrene standard): Mₙ = 9400, M_{w} = 21400, M_{w}/Mₙ = 2.28

### Example 9: Synthesis of non-yellowing thermoplastic polyurethane

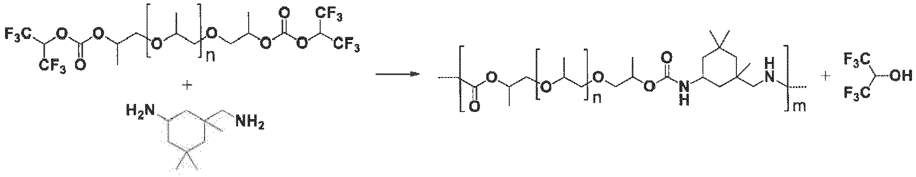

Polypropylene glycol bis(1,1,1,3,3,3-hexafluoroisopropyl carbonate) (0.39 g, 0.5 mmol) and isophoronediamine (0.09 g, 0.5 mmol) were added into a 50 mL round-bottom flask, and the mixture was stirred at 150°C for 1 hour. The reaction mixture was dried in vacuo at 50°C for 2 hours to obtain the target compound as pale yellow oil (yield: 97%, amount: 0.30 g, 0.48 mmol) .
¹H NMR (400 MHz, CDCl₃, 293 K): δ/ppm = 4.95-4.87(m, 2H, methine), 3.59-3.39(m, 22H, methylene), 2.94-2.88(br., 2H, methylene), 1.75-1.50(br., 6H), 1.24(d, J=5.6Hz, 6H, methyl), 1.16-1.14(d, J=6.0Hz, 18H, methyl), 1.07-0.83(m, 10H)
IR (ATR): 3326, 2970, 2931, 1699, 1530, 1460, 1374, 1302, 1240, 1098, 1027 cm⁻¹
Average molecular weight by HPLC (polystyrene standard): Mₙ = 8800, M_{w} = 16000, M_{w}/Mₙ = 1.82

### Example 10: Synthesis of hardly yellowing thermoplastic polyurethane

### (1) Synthesis of polypropylene glycol bis(2,2,3,3-tetrafluoropropyl carbonate) using inorganic base

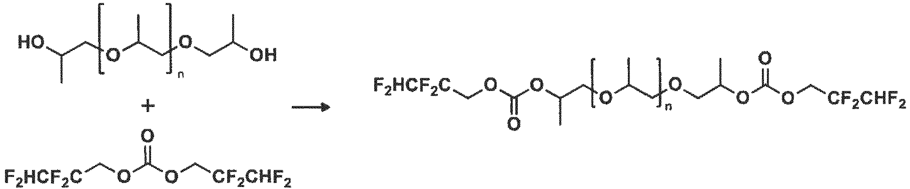

Bis(2,2,3,3-tetrafluoropropyl)carbonate (78.33 g, 270 mmol), propylene glycol 400 (36 mL, 90 mmol), potassium carbonate (1.3 g, 9 mmol) and acetonitrile (150 mL) as a solvent were added into a one-neck round-bottom flask, and the mixture was stirred at 50°C for 3 hours. Then, the solvent in the reaction mixture was evaporated under reduced pressure using an evaporator. Ethyl acetate, hexane and pure water were added thereto, and the organic phase and the aqueous phase were separated. The organic phase was dried over anhydrous sodium sulfate. The anhydrous sodium sulfate was removed by filtration, and the filtrate was concentrated under reduced pressure using an evaporator and dried in vacuo at 100°C for 2 hours to obtain the target compound as colorless transparent oil (yield: 85%, amount: 39.12 mmol).
¹H NMR (400 MHz, CDCl₃, 293 K): δ/ppm = 5.94(tt, J=53.0Hz, 4.0Hz, 2H, methine), 4.86-4.97(m, methylene), 4.61(tt, J=12.6Hz, 1.2Hz, 2H, methylene), 3.39-3.62(m, methylene and methine), 1.31(dd, J=6.8Hz, 3.2Hz, 6H, methyl), 1.13(m, methyl)
¹⁹F NMR (376 MHz, CDCl₃, 293 K): δ/ppm = -124.6, -138
IR (ATR): 2977, 2879, 1454, 1383, 1268, 1099, 992, 921, 786 cm⁻¹
FT-MS: m/z calculated for [M+Na]⁺ [C₁₄H₁₈F₈O₇(C₃H₆O)ₙ] 473.08 + 58.06n, found 763.28 (n=5), 821.32 (n=6), 879.36 (n=7), 937.40 (n=8), 995.44 (n=9), 1053 (n=10), 1111.52 (n=11)

### (2) Synthesis of polypropylene glycol bis(2,2,3,3-tetrafluoropropyl carbonate) using organic base

Bis(2,2,3,3-tetrafluoropropyl)carbonate (14.5 g, 50 mmol), propylene glycol 400 (4.0 g, 10 mmol) and triethylamine (0.2 g, 2 mmol) were added into a one-neck round-bottom flask, and the mixture was stirred at 90°C for 13 hours. Then, after the low-boiling component in the reaction mixture was concentrated under reduced pressure using an evaporator, the reaction mixture was dried in vacuo at 120°C for 1 hour to obtain the target compound as colorless transparent oil (yield: 94%).

### (3) Synthesis of polypropylene glycol bis(2,2,3,3-tetrafluoropropyl carbonate) using organic base

Polypropylene glycol 400 (0.4 g, 1.0 mmol), bis(2,2,3,3-tetrafluoropropyl)carbonate (0.72 g, 2.5 mmol) and 1,4-diazabicyclo(2,2,2)octane (hereinafter, described as DABCO) (0.01 g, 0.1 mmol) were added into a one-neck round-bottom flask, and the reaction mixture was stirred at 100°C for 13 hours. The obtained oily reaction mixture was dried in vacuo using an oil rotary pump at 100°C for 2 hours to evaporate the eliminated alcohol produced by the reaction and DABCO to obtain the target compound as transparent oil (yield: 96%).

### (4) Synthesis of hardly yellowing thermoplastic polyurethane

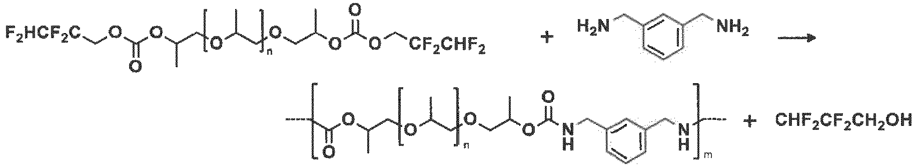

Polypropylene glycol bis(2,2,3,3-tetrafluoropropyl carbonate) (2.40 g, 3.36 mmol) and m-xylylenediamine (0.46 g, 3.36 mmol) were added into a 10 mL one-neck round-bottom flask, and the temperature was increased from 20°C to 120°C over 5 hours with stirring the reaction mixture under reduced pressure using a diaphragm pump. The reaction mixture was further stirred at 120°C for 2 hours. The pressure was returned to ordinary pressure and the reaction mixture was cooled to room temperature to quantitatively obtain the target compound as pale yellow oil.
¹H NMR (400 MHz, DMSO-d₆, 20°C): δ7.64(t, J=5.8Hz, 2H, NH), 7.24(t, J=8.0Hz, 1H, Phenyl), 7.11(s, 1H, Phenyl), 7.10(d, J=8.0Hz, 2H, Phenyl), 4.75(m, 2H, methine), 4.14(d, J=6.0Hz, 4H, methylene), 3.54-3.31(m, methine and methylene), 1.13(m., 6H, methyl), 1.04(d, J=6.0Hz, methyl)
Average molecular weight by HPLC (polystyrene standard): Mₙ = 3267, M_{w} = 4281, M_{w}/Mₙ = 1.28

### (5) Synthesis of hardly yellowing thermoplastic polyurethane

Polypropylene glycol bis(2,2,3,3-tetrafluoropropyl carbonate) (0.73 g, 1.0 mmol) and m-xylylenediamine (0.14 g, 1.0 mmol) were added into a one-neck round-bottom flask, and the mixture was stirred at 100°C for 13 hours. The obtained oily reaction mixture was dried in vacuo at 100°C for 1 hour using a diaphragm pump. It was confirmed by ¹H NMR spectrum that the corresponding polyurethane was produced.

The average molecular weight was determined by GPC; as a result, Mₙ was 4195, M_{w} was 5900 and M_{w}/Mₙ was 1.40. The fluoroalcohol eliminated by the reaction was not detected in the obtained polyurethane.

### Comparative example 3

Polyurethane was produced similarly to Example 10(5) except that the bis(phenyl carbonate) (0.64 g, 1.0 mmol) produced in Comparative example 1(1) was used in place of polypropylene glycol bis(2,2,3,3-tetrafluoropropyl carbonate) (0.73 g, 1.0 mmol). It was confirmed by ¹H NMR spectrum that the corresponding polyurethane was produced.

The average molecular weight was determined by GPC; as a result, Mₙ was 1318, M_{w} was 1715 and M_{w}/Mₙ was 1.30. In the obtained polyurethane, 5% of phenol remained to the total phenol eliminated by the reaction.

As the above-described results, phenol remained in polyurethane, though the polyurethane was dried in vacuo under high temperature. The reason why the polyurethane has smaller molecular weight and larger variation of molecular weight than the polyurethane of Example 10(5) may be that phenol as a by-product could not be removed and inhibited the polymerization reaction.

### Example 11: Synthesis of hardly yellowing thermoplastic polyurethane using solvent

Polypropylene glycol bis(2,2,3,3-tetrafluoropropyl carbonate) (1.0 g, 1.37 mmol), m-xylylenediamine and the solvent described in Table 1 were added into a 50 mL one-neck round-bottom flask, and the reaction mixture was heated and stirred in the conditions described in Table 1. It was confirmed by ¹H NMR spectrum that the corresponding polyurethane was produced, and the average molecular weight was determined by GPC. The result is shown in Table 1.

**Table 1**

| Diamine | Solvent | Reaction time | Reaction temperature | M_{w}, Mₙ, M_{w}/Mₙ |
|---|---|---|---|---|
| 179µL (1. 38mmol) | DMSO 392µL | 168h | 80°C | 3638, 2049, 1.78 |
| 179µL (1.38mmol) | DMF 427µL | 168h | 80°C | 2452, 1606, 1.53 |
| 199µL (1.53mmol) | THF 1332µL | 329h | 50°C | 5540, 3464, 1.60 |

Since there was no significant change in the average molecular weight of the obtained polyurethane in comparison with the polyurethane of Example 10(4), in which a solvent was not used, it was found that the reaction successfully proceeds even when a solvent is used.

### Example 12: Synthesis of hardly yellowing thermoplastic polyurethane using solvent

Polypropylene glycol bis(2,2,3,3-tetrafluoropropyl carbonate) (1.0 g, 1.37 mmol), THF (444 µL) and the diamine described in Table 1 were added into a 50 mL one-neck round-bottom flask, and the reaction mixture was stirred at 50°C for 332 hours. It was confirmed by ¹H NMR spectrum that the corresponding polyurethane was produced and the average molecular weight was determined by GPC. The result is shown in Table 2.

**Table 2**

| Diamine | M_{w}, Mₙ, M_{w}/Mₙ |
|---|---|
| 1,3-Cyclohexanedimethanamine 207µL (1.37mmol) | 1216, 849, 1.43 |
| 4,4'-Diaminocyclohexylmethane 0.288g (1.37mmol) | 3358, 2428, 1.39 |
| Isophoronediamine 253µL (1.37mmol) | 3271, 2407, 1.36 |

Since there was no significant change in the average molecular weight of the obtained polyurethane in comparison with the polyurethane of Example 10(4), in which a solvent was not used, it was found that the reaction successfully proceeds even when a solvent is used.

### Example 13: Synthesis of non-yellowing thermoplastic polyurethane

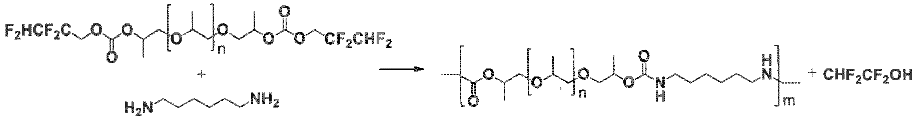

Polypropylene glycol bis(2,2,3,3-tetrafluoropropyl carbonate) (2.40 g, 3.36 mmol) and 1,6-hexamethylenediamine (0.40 g, 3.36 mmol) were added into a 20 mL one-neck round-bottom flask, and the temperature was increased from 20°C to 120°C over 5 hours with stirring the reaction mixture under reduced pressure using a diaphragm pump. The reaction mixture was further stirred at 120°C for 2 hours. The pressure was returned to ordinary pressure and the reaction mixture was cooled to room temperature to quantitatively obtain the target compound as pale yellow oil.
¹H NMR (400 MHz, DMSO-d₆, 20°C): δ7.07(s, 2H, NH), 4.73 (m, 2H, methine), 3.56-3.32(m, methine and methylene), 2.94(m, 4H, methylene), 1.36(br., methyl), 1.22(br., methyl), 1.11(m, methyl), 1.04(d, J=6.0Hz, methyl)
Average molecular weight by HPLC (polystyrene standard): Mₙ = 2690, M_{w} = 3886, M_{w}/Mₙ = 1.44

### Example 14: Synthesis of hardly yellowing thermoplastic polyurethane using solvent

Polypropylene glycol bis(2,2,3,3-tetrafluoropropyl carbonate) (1.0 g, 1.37 mmol), 1,6-hexamethylenediamine (0.11 g, 1.00 mmol) and the solvent described in Table 1, and further the base described in Table 1 in some cases were added into a 50 mL one-neck round-bottom flask, and the mixture was heated and stirred in the conditions described in Table 1. It was confirmed by ¹H NMR spectrum that the corresponding polyurethane was produced, and the average molecular weight was determined by GPC. The result is shown in Table 3.

**Table 3**

| Solvent | Base | Reaction time | Reaction temperature | M_{w}, Mₙ, M_{w}/Mₙ |
|---|---|---|---|---|
| DMF 5mmol | - | 24h | 80°C | 9516, 6143, 1.55 |
| THF 5mmol | - | 24h | 50°C | 7512, 6086, 1.23 |
| 1,4-Dioxane 5mmol | - | 24h | 80°C | 8582, 6753, 1.27 |
| DMSO 5mmol | - | 24h | 80°C | 10944, 9294, 1.18 |
| DMSO 5mmol | KF 0.137mmol | 357h | 80°C | 11783, 6190, 1.90 |

The average molecular weight of the polyurethane produced using a solvent was larger than that of Example 13 as the result shown in Table 3. The reason may be that the reaction was accelerated due to high flowability of the polymer. In addition, it was found that the reaction is further accelerated by using a base.

### Example 15

### (1) Synthesis of 1,6-hexamethylene bis(2,2,2-trifluoroethyl carbonate)

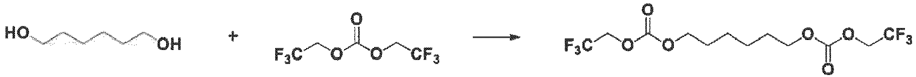

Bis(3,3,3-trifluoroethyl)carbonate (0.79 g, 3.5 mmol) and potassium carbonate (55 mg, 0.4 mmol) was added to acetonitrile (2 mL) to be mixed in a one-neck round-bottom flask. To the solution, 1,6-hexanediol (0.18 g, 1.5 mmol) was added. The mixture was stirred at 50°C for 3 hours. Dichloromethane and water were added to the reaction mixture, and the organic phase and the aqueous phase were separated. The organic phase was dried over anhydrous sodium sulfate. The anhydrous sodium sulfate was removed by filtration, and the filtrate was concentrated under reduced pressure to obtain the target compound as colorless oil (yield: 81%, amount: 0.45 g, 1.21 mmol).
¹H NMR (500 MHz, CDCl₃, 293 K): δ/ppm = 4.51(q, J=8.3Hz, 4H, methylene), 4.21(t, J=6.8Hz, 4H, methylene), 1.72(quin, J=7.0Hz, 4H, methylene), 1.43(quin, J=3.8Hz, 4H, methylene) ¹³C NMR (125 MHz, CDCl₃, 293 K): δ/ppm = 154.04, 122.58, 69.15, 63.32, 28.37, 25.25
¹⁹F NMR (376 MHz, CDCl₃, 293 K): δ/ppm = -74.26
IR (ATR): 2932, 1759, 1418, 1298, 1239, 1161, 972, 788 cm⁻¹
FAB-MS: m/z calculated for [M+H]⁺ (C₁₂H₁₆F₆O₆) 371.09, found 370.97

### Example 16

### (1) Synthesis of bis(hydroxyethyl)bisphenol A bis(2,2,2-trifluoroethyl carbonate)

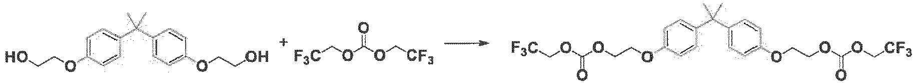

Bis(3,3,3-trifluoroethyl)carbonate (11.3 g, 50.0 mmol) and potassium carbonate (688 mg, 5.0 mmol) were added to acetonitrile (25 mL) to be mixed in a one-neck round-bottom flask. Bis(hydroxyethyl)bisphenol A (5.28 g, 16.7 mmol) was added to the obtained solution, and the mixture was heated and stirred at 50°C for 1 hour. Diethyl ether and water were added to the reaction mixture, and the organic phase and the aqueous phase were separated. The organic phase was dried over anhydrous sodium sulfate. The anhydrous sodium sulfate was removed by filtration, and the filtrate was concentrated under reduced pressure using an evaporator and further dried in vacuo at 80°C for 2 hours to obtain the target compound as brown oil (yield: 94%, amount: 8.9 g, 15.7 mmol).
¹H NMR (500 MHz, CDCl₃, 293 K): δ/ppm = 7.13(d, J=9.0Hz, 4H, phenyl), 6.80(d, J=9.0Hz, 4H, phenyl), 4.53(t, J=4.8Hz, 4H, methylene), 4.52(q, J=8.2Hz, 4H, methylene), 4.18(t, J=4.8Hz, 4H, methylene), 1.63(s, 6H, methyl)
¹³C NMR (125 MHz, CDCl₃, 293 K): δ/ppm = 156.05, 153.93, 143.80, 127.81, 122.46, 113.96, 67.33, 65.33, 63.49, 41.74, 30.98
¹⁹F NMR (376 MHz, CDCl₃, 293 K): δ/ppm = -74.16
IR (ATR): 2970, 1761, 1509, 1417, 1299, 1227, 1164, 987, 831 cm⁻¹
FAB-MS: m/z calculated for [M+H]⁺ (C₂₅H₂₆F₆O₈) 569.15, found 569.14

### Example 17

### (1) Synthesis of isosorbide bis(2,2,2-trifluoroethyl carbonate)

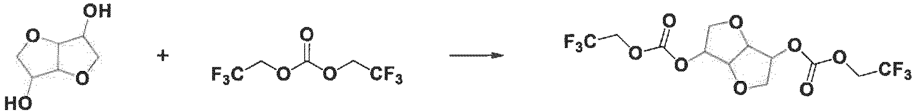

Bis(3,3,3-trifluoroethyl)carbonate (11.3 g, 50.0 mmol) and potassium carbonate (688 mg, 5.0 mmol) were added to acetonitrile (25 mL) to be mixed in a one-neck round-bottom flask. Isosorbide (2.44 g, 16.7 mmol) was added to the obtained solution, and the mixture was stirred at 50°C for 1 hour. Diethyl ether and water were added to the reaction mixture, and the organic phase and the aqueous phase were separated. The organic phase was dried over anhydrous sodium sulfate. The anhydrous sodium sulfate was removed by filtration, and the filtrate was concentrated under reduced pressure using an evaporator and further dried in vacuo at 80°C for 2 hours to obtain the target compound as brown oil (yield: 82%, amount: 5.5 g, 13.7 mmol).
¹H NMR (500 MHz, CDCl₃, 293 K): δ/ppm = 5.08-5.16(m, 2H), 4.87-4.99(m, 1H), 4.49-4.61(m, 5H), 4.06-4.12(m, 1H), 3.97-4.04(m, 2H), 3.87-3.94(m, 1H)
¹³C NMR (125 MHz, CDCl₃, 293 K): δ/ppm = 153.42, 153.11, 122.47, 122.39, 85.73, 82.12, 81.08, 77.83, 72.97, 70.77, 64.24, 63.66
¹⁹F NMR (376 MHz, CDCl₃, 293 K): δ/ppm = -74.25
IR (ATR): 2983, 2885, 1759, 1416, 1291, 1237, 1162, 1095, 990, 973, 778 cm⁻¹
FAB-MS: m/z calculated for [M+H]⁺ (C₁₂H₁₂F₆O₈) 399.04, found 399.00

### Example 18

### (1) Synthesis of 2,2'-thiodiethylene bis(1,1,1,3,3,3-hexafluoroisopropyl carbonate)

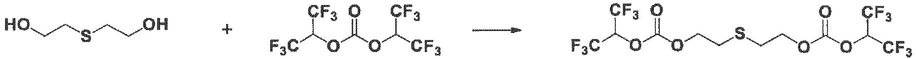

Bis(1,1,1,3,3,3-hexafluoropropane-2-yl)carbonate (1.81 g, 5 mmol), 2,2'-thiodiethanol (0.12 g, 1 mmol) and triethylamine (0.1 mmol, 13.8 µL) were added into a one-neck round-bottom flask, and the mixture was stirred at 90°C for 16 hours. Then, chloroform and water were added thereto, and the organic phase and the aqueous phase were separated. The organic phase was dried over anhydrous sodium sulfate. The anhydrous sodium sulfate was removed by filtration, and the filtrate was concentrated under reduced pressure using an evaporator and further dried in vacuo at 50°C for 3 hours to obtain the target compound as colorless oil (yield: 75%, amount: 0.38 g, 0.75 mmol).
¹H NMR (400 MHz, CDCl₃, 293 K): δ/ppm = 5.57(m, 2H, methine), 5.05-4.97(m, 2H, methine), 3.65-3.38(m, 19H, methylene and methine), 1.35-1.32(m, 6H, methyl), 1.14-1.10(m., 15H, methyl) ¹³C NMR (125 MHz, CDCl₃, 293 K): δ/ppm = 152.8, 120.3, 70.5, 69.0, 30.5
¹⁹F NMR (376 MHz, CDCl₃, 293 K): δ/ppm = -73.52
IR (ATR): 1774, 1382, 1296, 1253, 1238, 1197, 1189, 1140, 1106, 939, 929, 686, 677, 594, 565 cm⁻¹
FAB MS: m/z calculated for [M]⁺ (C₁₂H₁₀F₁₂O₆S) 510.00, found 509.93

### Example 19

### (1) Synthesis of poly tetramethylene ether glycol bis(1,1,1,3,3,3-hexafluoroisopropyl carbonate)

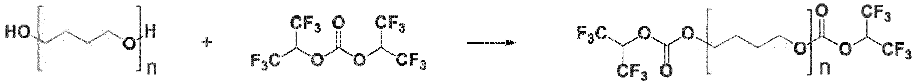

Bis(1,1,1,3,3,3-hexafluoropropane-2-yl)carbonate (3.62 g, 10 mmol), poly tetramethylene ether glycol (PTMG 1500) (1.5 g, 1 mmol) and triethylamine (0.1 mmol, 13.8 µL) were added to a one-neck round-bottom flask, and the mixture was stirred at 90°C for 16 hours. Then, chloroform and water were added thereto, and the organic phase and the aqueous phase were separated. The organic phase was dried over anhydrous sodium sulfate. The anhydrous sodium sulfate was removed by filtration, and the filtrate was concentrated under reduced pressure and further dried in vacuo at 60°C for 1 hour to quantitatively obtain the target compound as white solid (1.93 g, 1.0 mmol).
¹H NMR (400 MHz, CDCl₃, 293 K): δ/ppm = 5.56(m, 2H, methine), 4.32(t, J=5.2Hz, 4H, methylene), 3.45-3.36(m, 82H, methylene), 1.83(m, 4H, methylene), 1.68-1.57(m, 82H, methylene)
¹⁹F NMR (376 MHz, CDCl₃, 293 K): δ/ppm = -73.52
IR (ATR): 2942, 2862, 1777, 1371, 1253, 1199, 1106, 1065, 1012, 996, 617, 607, 594, 582, 567, 554 cm⁻¹
FT-MS: m/z calculated for [M+Na]⁺ [C₁₂H₁₀F₁₂O₆(C₄H₈O)ₙ] 501.02 + 72.07n, found 789.25 (n=4), 861.31 (n=5), 933.36 (n=6), 1005.42 (n=7), 1077.48 (n=8), 1149.53 (n=9), 1221.59 (n=10), 1365.71 (n=11), 1365.71 (n=12), 1437.76 (n=13), 1509.82 (n=14), 1581.88 (n=15), 1653.94 (n=16), 1725.99 (n=17), 1798.05 (n=18), 1870.11 (n=19)

### Example 20

### (1) Synthesis of 1,1'-thiodiethylene bis(2,2,3,3-tetrafluoropropyl carbonate)

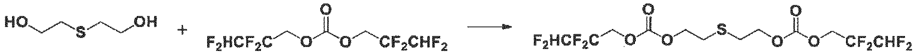

Bis(2,2,3,3-tetrafluoropropyl)carbonate (16 mL, 90 mmol), 1,1'-thiodiethanol (3.7 g, 30 mmol), potassium carbonate (0.42 g, 3 mmol) and acetonitrile (50 mL) as a solvent were added into a one-neck round-bottom flask, and the mixture was stirred at 50°C for 3 hours. Then, the reaction mixture was concentrated under reduced pressure using an evaporator. Dichloromethane and pure water were added thereto, and the organic phase and the aqueous phase were separated. The organic phase was dried over anhydrous sodium sulfate. The anhydrous sodium sulfate was removed by filtration, and the filtrate was concentrated under reduced pressure using an evaporator. The residue was subjected to distillation using a glass tube oven to obtain the fraction of 170°C as the target compound of colorless transparent oil (yield: 15%, amount: 1.97 g, 8.67 mmol).
¹H NMR (400 MHz, CDCl₃, 293 K): δ/ppm = 5.92(tt, J=53.0Hz, 4.0Hz, 2H, methine), 4.54(tt, J=12.6Hz, 1.2Hz, 4H, methylene), 4.36(t, J=6.8Hz, 4H, methylene), 2.86(t, J=6.8Hz, 4H, methylene)
¹⁹F NMR (376 MHz, CDCl₃, 293 K): δ/ppm = -124.2, -137.6
IR (ATR): 1760, 1452, 1267, 1202, 984, 835, 636, 574 cm⁻¹ FAB-MS: m/z calculated for [M+H]⁺ (C₁₁H₁₂F₈O₆S) 411.01, found 411.04

### Example 21

### (1) Synthesis of 2,2,3,3,4,4-hexafluoro-1,5-pentamethylene bis(1,1,1,3,3,3-hexafluoroisopropyl carbonate)

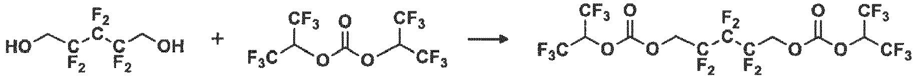

BHFC (24 mmol, 5.3 mL), 2,2,3.3.4.4-hexafluoro-1,5-pentanediol (1,5-HFPDL) (4.0 mmol, 0.84 g), pyridine (0.40 mmol, 32 µL) and acetonitrile (2 mL) as a solvent were added to a 50 mL round-bottom flask, and the mixture was stirred at 20°C for 6 hours. Then, the solvent was evaporated under reduced pressure, and the residue was heated at 50°C under reduced pressure to be dried to obtain the target compound as colorless liquid (amount: 2.2 g, 3.6 mmol, yield: 90%).
¹H NMR (400 MHz, CDCl₃, 293 K): δ5.56 (sep, J=5.8Hz, 2H, CH), 4.77(t, J=13Hz, 4H, CH₂)
¹³C NMR (125 MHz, CDCl₃, 293 K): δ152.4, 120.0(q, J=283Hz), 113.8(tt, J=257, 31Hz), 110.9(tt, J=261, 33Hz), 71.2(sep), 64.6(t, J=28Hz)
¹⁹F NMR (376 MHz, CDCl₃, 293 K, C₆F₆ as external standard): δ-73.63(s, 12F, CF₃), -120.20(s, 4F, CF₂), -125.66(s, 2F, CF₂) IR (ATR): 2985, 1787, 1384, 1254, 1200, 1152, 1111, 1045, 985, 906, 777, 689 cm⁻¹

### (2) Synthesis of polyurethane

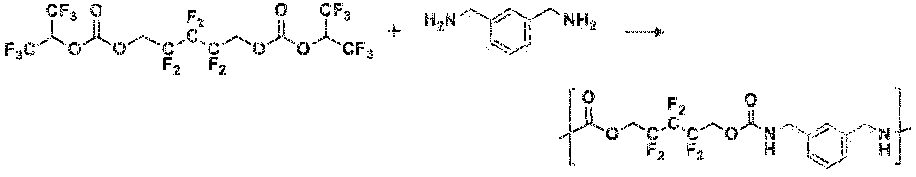

Into a 7 mL test tube, 2,2,3,3,4,4-hexafluoro-1,5-pentamethylene bis(1,1,1,3,3,3-hexafluoroisopropyl carbonate) (0.30 g, 0.50 mmol) and m-xylylenediamine (73 µL, 0.53 mmol) were added. The mixture was stirred at 100°C for 2 hours. Then, appropriate amounts of methanol and hexane were added to the reaction mixture, and the precipitated solid was collected by suction filtration. The solid was dried in vacuo at 50°C for 2 hours to obtain the target compound as pale yellow solid (amount: 0.14 g, 0.35 mmol, yield: 71%).
¹H NMR (400 MHz, acetone-d₆, 293 K): δ7.31-7.21(m, 4H, CHAr), 4.69(t, J=14Hz, 4H, CH₂), 4.34 (s, 4H, CH₂)
¹³C NMR (125 MHz, acetone-d₆, 293 K): δ155.8, 140.3, 129.5, 127.2, 126.9, 116.2(tt), 112.3(tt), 60.5(t, J=25Hz), 45.3
¹⁹F NMR (376 MHz, acetone-d₆, 293 K, C₆F₆ as external standard): δ-120.87 (s, 4F, CF₂), -127.08 (s, 2F, CF₂)
IR (ATR): 3322, 1703, 1529, 1457, 1260, 1149, 1055, 967, 776 cm⁻¹
Average molecular weight by HPLC: Mₙ = 6500, M_{w} = 11100, M_{w}/Mₙ = 1.7

### Example 22: Synthesis of polyurethane

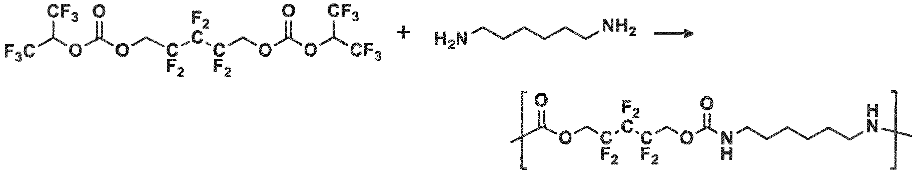

Into a 7 mL test tube, 2,2,3,3,4,4-hexafluoro-1,5-pentamethylene bis(1,1,1,3,3,3-hexafluoroisopropyl carbonate) (0.30 g, 0.50 mmol) and 1,6-hexamethylenediamine (55 mg, 0.48 mmol) were added. The mixture was stirred at 100°C for 2 hours. Then, appropriate amounts of acetone and hexane were added to the reaction mixture, and the precipitated solid was collected by suction filtration. The solid was dried in vacuo at 50°C for 2 hours to obtain the target compound as white solid (amount: 0.18 g, 0.48 mmol, yield: 95%).
¹H NMR (400 MHz, acetone-d₆, 293 K): δ6.74(t, J=5.6Hz, 2H, NH), 4.65(t, J=14Hz, 4H, CH₂), 3.17(td, J=6.6, 6.6Hz, 4H, CH₂), 1.54(t, J=6.6Hz, 4H, CH₂), 1.37 (m, 4H, CH₂)
¹³C NMR (125 MHz, acetone-d₆, 293 K): δ155.6, 116.2(tt, J=220, 26Hz), 112.3(tt), 60.2(t, J=25Hz), 41.7, 30.4, 27.0
¹⁹F NMR (376 MHz, acetone-d₆, 293 K, C₆F₆ as external standard): δ-120.98(br. , 4F, CF₂), -127.25(br., 2F, CF₂)
IR (ATR): 3331, 2938, 2864, 1703, 1535, 1257, 1151, 978, 896, 773 cm⁻¹
Average molecular weight by HPLC: Mₙ = 6500, M_{w} = 11300, M_{w}/Mₙ = 1.7

### Example 23: Synthesis of polyurethane

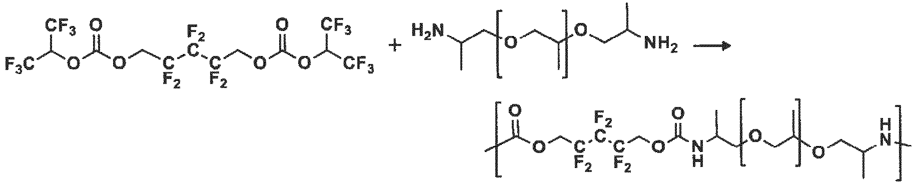

Into a 7 mL test tube, 2,2,3,3,4,4-hexafluoro-1,5-pentamethylene bis(1,1,1,3,3,3-hexafluoroisopropyl carbonate) (0.30 g, 0.50 mmol) and polypropylene glycol diamine (PPGDA, average molecular weight: 430) (0.22 g, 0.50 mmol) were added. The mixture was stirred at 100°C for 2 hours. Then, the reaction mixture was dried in vacuo at 50°C for 3 hours to obtain the target compound as yellow viscous solid (amount: 0.36 g, 0.50 mmol, yield: >99%).
¹H NMR (400 MHz, acetone-d₆, 293 K): δ6.58(br., 2H, NH), 4.68-4.64 (br., 4H, CH₂), 3.77(m, 2H, CH), 3.60-3.36(m, 19H, CH+CH₂), 1.18(br., 6H, CH₃),1.10(br., 15H, CH₃)
¹³C NMR (125 MHz, acetone-d₆, 293 K): δ154.9, 116.2(tt, J=255, 30Hz), 112.2(tt), 76.5-72.7(m), 60.2(t, J=25Hz), 48.5-48.2(m), 17.7
¹⁹F NMR (376 MHz, acetone-d₆, 293 K, C₆F₆ as external standard) : δ-120.89(br.), -127.17(br.)
IR (ATR): 3330, 2974, 1873, 1721, 1534, 1428, 1375, 1240, 1098, 927, 769 cm⁻¹
Average molecular weight by HPLC: Mₙ = 6600, M_{w} = 15000, M_{w}/Mₙ = 2.3

### Example 24: Synthesis of polyurethane

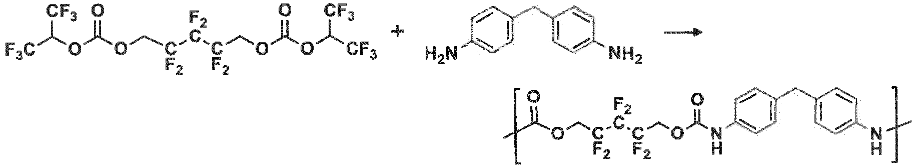

Into a 20 mL round-bottom flask, 2,2,3,3,4,4-hexafluoro-1,5-pentamethylene bis(1,1,1,3,3,3-hexafluoroisopropyl carbonate) (0.30 g, 0.50 mmol), 4,4'-diaminodiphenylmethane (0.50 mmol, 99 mg) and THF (2 mL) as a solvent were added. The mixture was stirred at 60°C for 27 days. Samples were taken from the reaction solution approximately every 3 days. The progress of the reaction was confirmed by mixing the samples with deuterated acetone and analyzing the mixture by ¹H NMR. Then, appropriate amounts of THF and hexane were added to the reaction mixture, and the precipitated solid was collected by suction filtration. The solid was dried in vacuo at 50°C for 3 hours to obtain the target compound as pale brown solid (amount: 0.21 g, 0.46 mmol, yield: 91%).
¹H NMR (400 MHz, DMSO-d₆, 293 K): δ10.05(s, 2H, NH), 7.38(d, J=7.6Hz, 4H, CHAr), 7.14(d, J=7.6Hz, 4H, CHAr), 4.82(t, J=15Hz, 4H, CH₂), 3.82 (s, 2H, CH₂)
¹³C NMR (125 MHz, DMSO-d₆, 293 K): δ151.8, 136.2, 136.1, 128.9, 118.6, 115.0(tt), 113.0(tt), 59.0(t, J=21Hz), 39.7
¹⁹F NMR (376 MHz, DMSO-d₆, 293 K, C₆F₆ as external standard): δ-119.20(br., 4F, CF₂), -125.40(br., 2F, CF₂)
IR (ATR): 3334, 1716, 1598, 1536, 1415, 1236, 1158, 1112, 992, 814, 762 cm⁻¹
Average molecular weight by HPLC: Mₙ = 6300, M_{w} = 13400, M_{w}/Mₙ = 2.1

### Example 25

### (1) Synthesis of 2,2,3,3,4,4,5,5-octafluoro-1,6-hexamethylene bis(1,1,1,3,3,3-hexafluoroisopropyl carbonate)

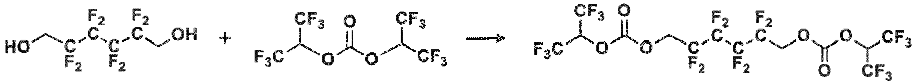

BHFC (54 mmol, 11.9 mL), 2,2,3,3,4,4,5,5-octafluoro-1,6-hexanediol (9.0 mmol, 2.36 g), pyridine (1.35 mmol, 108 µL) and acetonitrile (4 mL) as a solvent were added to a 50 mL round-bottom flask. The mixture was stirred at 20°C for 6 hours. Then, appropriate amounts of 1 M hydrochloric acid and hydrofluoroether ("Novec^{™} 7100" manufactured by 3M) were added to the reaction mixture, and the organic phase and the aqueous phase were separated. The organic phase was dried over anhydrous sodium sulfate. The solvent was evaporated under reduced pressure from the obtained solution, and the residue was dried in vacuo at 50°C for 2 hours to obtain the target compound as white solid (amount: 5.6 g, 8.7 mmol, yield: 96%).
¹H NMR (400 MHz, CDCl₃, 293 K): δ5.56(sep, J=6.0Hz, 2H, CH), 4.77(t, J=13Hz, 4H, CH₂)
¹³C NMR (100 MHz, CDCl₃, 293 K): 5152.38, 119.94, 115.73, 113.70, 71.17, 64.42
¹⁹F NMR (376 MHz, CDCl₃, 293 K): δ-73.49(s, 12F, CF₃), - 119.99(s, 4F, CF₂), -123.65(s, 4F, CF₂)
IR (ATR): 2997, 1790, 1443, 1411, 1390, 1371, 1322, 1293, 1255, 1234, 1203, 1179, 1111, 1044, 1015, 943, 907, 868, 775, 734 cm⁻¹

### (2) Synthesis of polyurethane

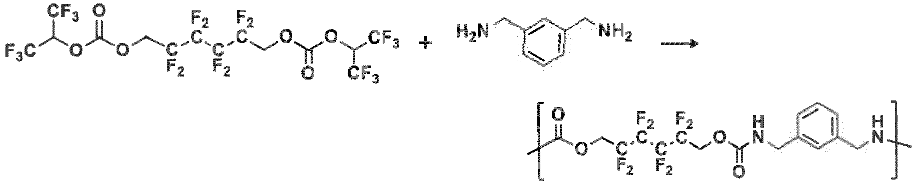

Into a 7 mL test tube, 2,2,3,3,4,4,5,5-octafluoro-1,6-hexamethylene bis(1,1,1,3,3,3-hexafluoroisopropyl carbonate) containing 16.7% of carbonate oligomer (0.33 g, 0.42 mmol) and m-xylylenediamine (66 µL, 0.50 mmol) were added. The mixture was stirred at 150°C for 1 hour. Then, appropriate amounts of acetone and hexane were added to the reaction mixture, and the precipitated solid was collected by suction filtration. The solid was dried in vacuo at 50°C for 1.5 hours to obtain the target compound as white solid (amount: 0.19 g, 0.38 mmol, yield: 86%).
¹H NMR (400 MHz, acetone-d₆, 293 K): δ7.32-7.19(m, 4H, CHAr), 4.72(t, J=14.4Hz, 4H, CH₂), 4.37 (d, J=6.4Hz, 4H, CH₂)
¹³C NMR (125 MHz, acetone-d₆, 293 K): δ155.77, 140.30, 129.44, 127.27, 126.97, 116.16, 112.20, 60.47, 45.45
¹⁹F NMR (376 MHz, acetone-d₆, 293 K): δ-120.66(s, 4F, CF₂), - 124.36(s, 4F, CF₂)
IR (ATR): 3331, 3060, 2973, 2928, 2881, 1698, 1538, 1350,
1256, 1173, 1146, 1118, 1056, 961, 870, 771 cm⁻¹
Average molecular weight by HPLC: Mₙ = 9500, M_{w} = 18200, M_{w}/Mₙ = 1.5

### (3) Synthesis of polyurethane

Into a 50 mL round-bottom flask, 2,2,3,3,4,4,5,5-octafluoro-1,6-hexamethylene bis(1,1,1,3,3,3-hexafluoroisopropyl carbonate) containing 9.5% of carbonate oligomer (3.25 g, 4.53 mmol), m-xylylenediamine (587 µL, 4.53 mmol) and tetrahydrofuran (12.5 mL) as a solvent were added. The mixture was stirred at 65°C for 2 hours. Then, the solvent was evaporated under reduced pressure, and the residue was dried in vacuo at 50°C for 2 hours to obtain the target compound as white solid (amount: 2.39 g, 4.53 mmol, yield: 99%).
Average molecular weight by HPLC: Mₙ = 7900, M_{w} = 13900, M_{w}/Mₙ = 1.8

### (4) Synthesis of polyurethane

Into a 50 mL round-bottom flask, 2,2,3,3,4,4,5,5-octafluoro-1,6-hexamethylene bis(1,1,1,3,3,3-hexafluoroisopropyl carbonate) containing 10.7% of carbonate oligomer (2.18 g, 3.0 mmol), m-xylylenediamine (356 µL, 2.74 mmol) and tetrahydrofuran (10 mL) as a solvent were added. The mixture was stirred at room temperature for 34 hours. Then, the solvent was evaporated under reduced pressure, and the residue was dried in vacuo at 50°C for 2 hours to obtain the target compound as white solid containing unreacted carbonate and m-xylylenediamine.
Average molecular weight by HPLC: Mₙ = 5700, M_{w} = 6600, M_{w}/Mₙ = 1.2

### Example 26: Synthesis of polyurethane

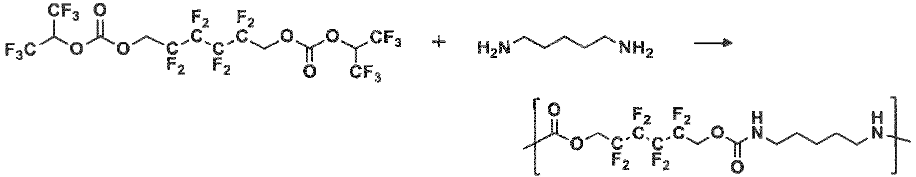

Into a 100 mL round-bottom flask, 2,2,3,3,4,4,5,5-octafluoro-1,6-hexamethylene bis(1,1,1,3,3,3-hexafluoroisopropyl carbonate) containing 7.8% of carbonate oligomer (0.65 g, 0.92 mmol) and 1,5-pentamethylenediamine (108 µL, 0.92 mmol) were added. The mixture was stirred at 150°C for 1 hour. Then, the reaction mixture was mixed with tetrahydrofuran, the solvent was evaporated under reduced pressure, and the residue was dried in vacuo at 50°C for 2 hours to obtain the target compound as brown solid (amount: 0.44 g, 0.83 mmol, yield: 90%).
¹H NMR (400 MHz, acetone-d₆, 293 K): δ6.73(br., 2H, NH), 4.68(t, J=14Hz, 4H, CH₂), 3.17(q, J=6.6Hz, 4H, CH₂), 1.57(qin, J=7.4Hz, 4H, CH₂), 1.40(m, 4H, CH₂)
¹³C NMR (125 MHz, acetone-d₆, 293 K): 5155.49, 116.19, 112.20, 60.22, 41.72, 24.43
¹⁹F NMR (376 MHz, acetone-d₆, 293 K): δ-120.73(br., 4F, CF₂), - 124.41(br., 4F, CF₂)
IR (ATR): 3354, 2945, 2868, 1703, 1533, 1455, 1260, 1227, 1170, 1146, 1122, 1045, 974, 870, 773 cm⁻¹
Average molecular weight by HPLC: Mₙ = 11600, M_{w} = 22100, M_{w}/Mₙ = 1.9

### Example 27: Synthesis of polyurethane

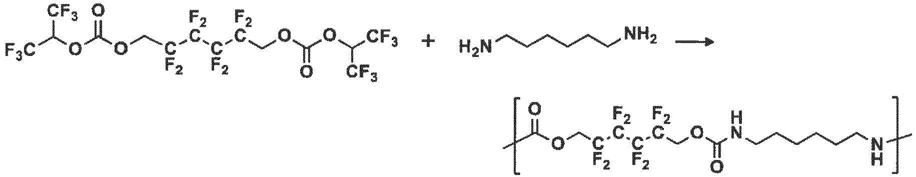

(1) Into a 100 mL round-bottom flask, 2,2,3,3,4,4,5,5-octafluoro-1,6-hexamethylene bis(1,1,1,3,3,3-hexafluoroisopropyl carbonate) containing 9.5% of carbonate oligomer (0.65 g, 0.92 mmol) and 1,6-hexamethylenediamine (0.105 g, 0.92 mmol) were added. The mixture was stirred at 150°C for 1 hour. Then, the reaction mixture was mixed with tetrahydrofuran, the solvent was evaporated under reduced pressure, and the residue was dried in vacuo at 50°C for 2 hours to obtain the target compound as white solid (amount: 0.46 g, 0.85 mmol, yield: 92%).
   ¹H NMR (400 MHz, acetone-d₆, 293 K): δ6.72(t, J=4.8Hz, 2H, NH), 4.68(m, J=14Hz, 4H, CH₂), 3.17(q, J=6.6Hz, 4H, CH₂), 1.54(m,
   4H, CH₂), 1.37(m, 4H, CH₂)
   ¹³C NMR (100 MHz, acetone-d₆, 293 K): δ155.47, 116.19, 112.19, 60.19, 41.72, 26.99
   ¹⁹F NMR (376 MHz, acetone-d₆, 293 K): δ-120.77(br., 4F, CF₂), - 124.45(br., 4F, CF₂)
   IR (ATR): 3344, 2942, 2863, 1791, 1702, 1535, 1259, 1174, 1147, 1119, 1063, 975, 870, 838, 773, 755 cm⁻¹
   Average molecular weight by HPLC: Mₙ = 11000, M_{w} = 17600, M_{w}/Mₙ = 1.6
(2) Into a 20 mL round-bottom flask, 2,2,3,3,4,4,5,5-octafluoro-1,6-hexamethylene bis(1,1,1,3,3,3-hexafluoroisopropyl carbonate) containing 10.7% of carbonate oligomer (2.18 g, 3.0 mmol), 1,6-hexamethylenediamine (0.349 g, 3.0 mmol) and tetrahydrofuran (15 mL) as a solvent were added. The mixture was stirred at room temperature for 5 hours. Then, the solvent was evaporated, and the residue was dried in vacuo at 50°C for 2 hours to obtain the target compound as white solid (amount: 1.43 g, 3.0 mmol, yield: >99%).
   Average molecular weight by HPLC: Mₙ = 7200, M_{w} = 12400, M_{w}/Mₙ = 1.7

### Example 28: Synthesis of polyurethane

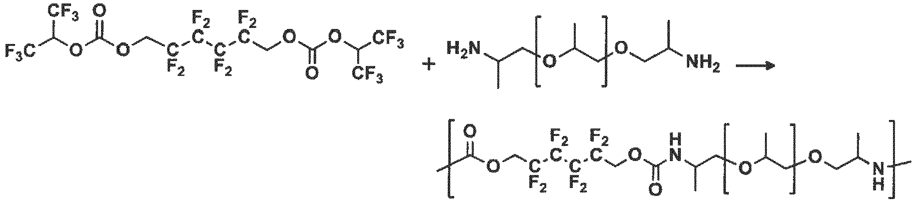

(1) Into a 7 mL test tube, 2,2,3,3,4,4,5,5-octafluoro-1,6-hexamethylene bis(1,1,1,3,3,3-hexafluoroisopropyl carbonate) containing 17.5% of carbonate oligomer (0.38 g, 0.48 mmol), polypropylene glycol diamine (PPGDA, average molecular weight: 400) (0.20 g, 0.50 mmol) and tetrahydrofuran (1 mL) as a solvent were added. The mixture was stirred at 30°C for 1 hour. Then, the solvent was evaporated under reduced pressure, and the residue was dried in vacuo at 60°C for 7 hours to obtain the target compound as colorless transparent viscous liquid (amount: 0.35 g, 0.50 mmol, yield: 98%).
   ¹H NMR (400 MHz, acetone-d₆, 293 K): δ6.60-6.54(br., 2H, NH), 4.72-4.68(br., 4H, CH₂), 3.80(m, 2H, CH), 3.63-3.36(m, 19H, CH+CH₂), 1.18(br., 6H, CH₃), 1.11(br., 15H, CH₃)
   ¹³C NMR (125 MHz, acetone-d₆, 293 K): δ154.91, 116.19, 112.19, 76.54-72.70, 60.35, 48.39, 17.69
   ¹⁹F NMR (376 MHz, acetone-d₆, 293 K): δ-120.64(br.), - 124.33(br.)
   IR (ATR): 3319, 2976, 2935, 2875, 1782, 1732, 1531, 1456, 1377, 1175, 1100, 1020, 986, 929, 870, 840, 771 cm⁻¹
   Average molecular weight by HPLC: Mₙ = 8300, M_{w} = 13600, M_{w}/Mₙ = 1.6
(2) Into a 10 mL round-bottom flask, 2,2,3,3,4,4,5,5-octafluoro-1,6-hexamethylene bis(1,1,1,3,3,3-hexafluoroisopropyl carbonate) (0.65 g, 1.0 mmol), polypropylene glycol diamine (PPGDA, average molecular weight: 230) (0.23 g, 1.0 mmol) and tetrahydrofuran (2 mL) were added. The mixture was stirred at 20°C for 2 hours. Then, the solvent was evaporated under reduced pressure, and the residue was dried in vacuo at 50°C for 2 hours to obtain the target compound as pale yellow transparent viscous liquid (amount: 0.68 g, 1.0 mmol, yield: >99%, containing eliminated HFIP).
   ¹H NMR (400 MHz, acetone-d₆, 293 K): δ6.64-6.52(br., 2H, NH), 4.75-4.62(br., 4H, CH₂), 3.87-3.75(br., 2H, CH), 3.58-3.36(m, 19H, CH+CH₂), 1.18(br., 6H, CH₃), 1.10(br., 15H, CH₃)
   ¹³C NMR (125 MHz, acetone-d₆, 293 K): δ154.92, 116.19, 112.19, 76.54-72.70, 60.35, 48.34, 17.79
   ¹⁹F NMR (376 MHz, acetone-d₆, 293 K): δ-120.60(br.), - 124.31(br.)
   IR (ATR): 3327, 2977, 2937, 2878, 2360, 1715, 1531, 1456, 1378, 1287, 1226, 1173, 1125, 1100, 983, 894, 870, 841, 771 cm⁻¹
   Average molecular weight by HPLC: Mₙ = 13100, M_{w} = 22400, M_{w}/Mₙ = 1.7

### Example 29

### (1) Synthesis of perfluoropolyether (1,1,1,3,3,3-hexafluoroisopropylcarbonate)

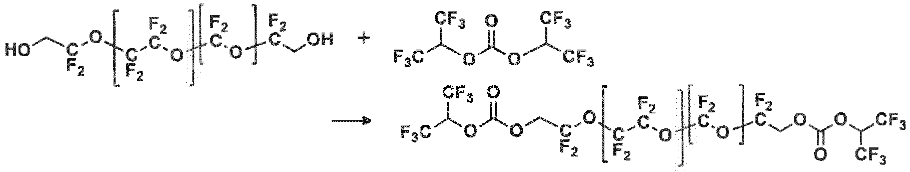

BHFC (4.8 mmol, 1.1 mL), perfluoropolyether (HOCH₂CF₂O(CF₂CF₂O)ₚ(CF₂O)_{q}CF₂CH₂OH, "Fomblin (registered trademark) D2" manufactured by Solvay) (0.80 mmol, 1.2 g) and pyridine (0.16 mmol, 13 µL) were added into a 50 mL round-bottom flask. The mixture was stirred at 70°C for 2 hours. Then, appropriate amounts of 1 M hydrochloric acid and hydrofluoroether ("Novec^{™} 7100" manufactured by 3M) were added to the reaction mixture, and the organic phase and the aqueous phase were separated. The organic phase was dried over anhydrous sodium sulfate. The solution was concentrated under reduced pressure, and the residue was dried in vacuo at 75°C for 1 hour to obtain the target compound as colorless liquid (amount: 1.2 g, 0.62 mmol, yield: 78%).
¹H NMR (400 MHz, CDCl₃, 293 K): δ5.54(br., 2H, CH), 4.63(br., 4H, CH₂)
¹⁹F NMR (376 MHz, CDCl₃, 293 K): δ-53.45(m, 12F, CF₂), -73.69(s, 12F, CF₃), -77.79(m, 4F, CF₂), -88.88, -91.97(m, 32F, CF₂)
IR (ATR): 1796, 1386, 1190, 1048, 996, 908 cm⁻¹

### (2) Synthesis of polyurethane

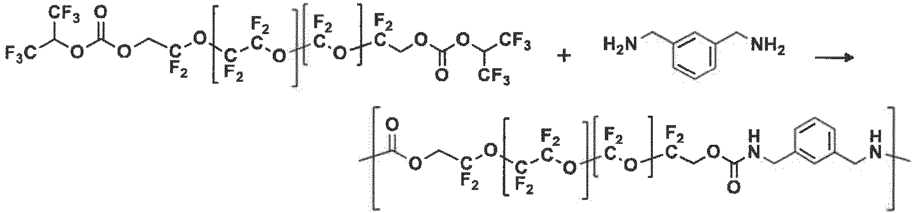

Perfluoropolyether bis(1,1,1,3,3,3-hexafluoroisopropyl carbonate) (0.38 g, 0.20 mmol) and m-xylylenediamine (24 µL, 0.18 mmol) were added into a 7 mL test tube, and the mixture was stirred at 100°C for 2 hours. Then, the reaction mixture was concentrated in vacuo at 50°C for 2 hours to obtain the target compound as colorless viscous liquid (amount: 0.37 g, 0.20 mmol, yield: >99%).
¹H NMR (400 MHz, CDCl₃, 293 K): δ7.21-7.05(m, 4H, CHAr), 4.46(br., 4H, CH₂), 4.35(br., 4H, CH₂)
¹⁹F NMR (376 MHz, CDCl₃, 293 K): δ-51.67 to -56.67 (m, 12F, CF₂),
-78.02 to -81.05(m, 4F, CF₂), -88.8 to -92.02(m, 32F, CF₂) IR (ATR): 3351, 1719, 1541, 1185, 1052, 697 cm⁻¹
Average molecular weight by HPLC: Mₙ = 6400, M_{w} = 12600, M_{w}/Mₙ = 2.0

### Example 30: Synthesis of polyurethane

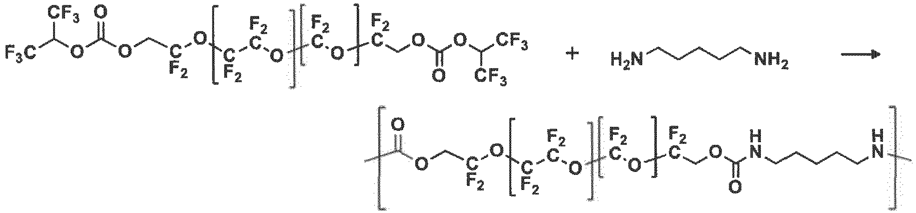

Perfluoropolyether bis(1,1,1,3,3,3-hexafluoroisopropyl carbonate) (0.38 g, 0.20 mmol) and 1,5-pentamethylenediamine (23 µL, 0.18 mmol) were added into a 7 mL test tube, and the mixture was stirred at 100°C for 2 hours and further at 120°C for 30 minutes in a reduced pressure condition using an oil pump. Then, the reaction mixture was concentrated in vacuo at 50°C for 2 hours to obtain the target compound as pale yellow viscous solid (amount: 0.34 g, 0.20 mmol, yield: >99%).
¹H NMR (400 MHz, CDCl₃, 293 K): δ4.45(br., 4H, CH₂), 3.24(br., 4H, CH₂), 1.38(br., 6H, CH₂)
¹⁹F NMR (376 MHz, CDCl₃, 293 K): δ-51.76 to -56.61(m, 12F, CF₂), -77.92 to -81.13(m, 4F, CF₂), -88.87 to -91.85(m, 32F, CF₂)
IR (ATR): 3332, 2942, 1720, 1521, 1186, 1059, 815, 695 cm⁻¹
Average molecular weight by HPLC: Mₙ = 6400, M_{w} = 13000, M_{w}/Mₙ = 2.0

### Example 31: Synthesis of polyurethane

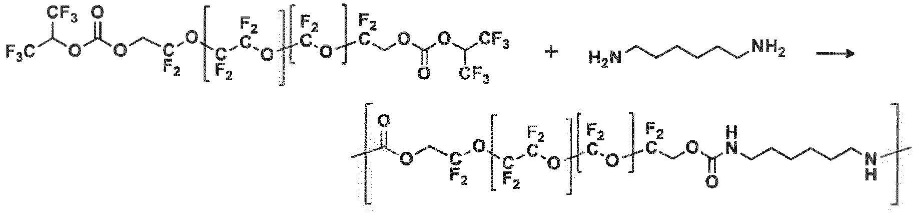

Perfluoropolyether bis(1,1,1,3,3,3-hexafluoroisopropyl carbonate) (0.38 g, 0.20 mmol) and 1,6-hexamethylenediaimne (23 µL, 0.18 mmol) were added to a 7 mL test tube, and the mixture was stirred at 100°C for 2 hours. Then, the reaction mixture was concentrated in vacuo at 80°C for 1 hour to obtain the target compound as colorless viscous liquid (amount: 0.31 g, 0.19 mmol, yield: 94%).
¹H NMR (400 MHz, CDCl₃, 293 K): δ5.73(br., 2H, NH), 4.48(br., 4H, CH₂), 3.25(br., 4H, CH₂), 1.45-1.36(br., 4H, CH₂), 1.24-1.19(br., 4H, CH₂)
¹⁹F NMR (376 MHz, CDCl₃, 293 K): δ-51.84 to -56.60(m, 12F, CF₂), -75.41 to -81.15(m, 4F, CF₂), -88.89 to -91.90(m, 32F, CF₂)
IR (ATR): 3332, 2944, 1721, 1523, 1186, 1058, 850, 683 cm⁻¹
Average molecular weight by HPLC: Mₙ = 8300, M_{w} = 17100, M_{w}/Mₙ = 2.1

### Example 32: Synthesis of polyurethane

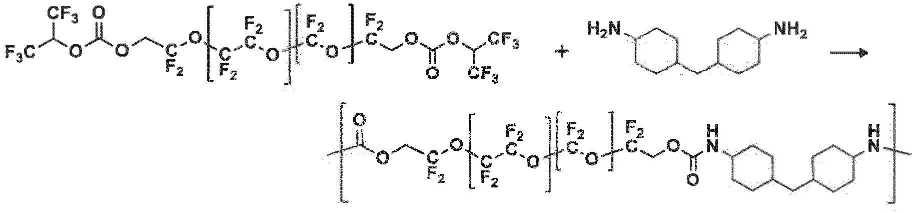

Perfluoroether bis(1,1,1,3,3,3-hexafluoroisopropyl carbonate) (0.38 g, 0.20 mmol) and 4,4'-methylene bis(cyclohexylamine) (23 µL, 0.18 mmol) were added into a 7 mL test tube, and the mixture was stirred at 100°C for 2 hours. Then, the reaction mixture was concentrated in vacuo at 80°C for 1 hour to obtain the target compound as colorless viscous liquid (amount: 0.31 g, 0.19 mmol, yield: 94%).
¹H NMR (400 MHz, CDCl₃, 293 K): δ4.43(br., 4H, CH₂), 3.42(br., 2H, CH), 2.01-0.98(m, 20H, CH₂)
¹⁹F NMR (376 MHz, CDCl₃, 293 K): δ-51.69 to -56.41(m, 12F, CF₂), -77.91 to -80.74(m, 4F, CF₂), -88.84 to -91.91(m, 32F, CF₂)
IR (ATR): 3332, 2944, 1721, 1523, 1186, 1058, 850, 683 cm⁻¹
Average molecular weight by HPLC: Mₙ = 9400, M_{w} = 20500, M_{w}/Mₙ = 2.2

### Example 33: Synthesis of polyurethane

### (1) Synthesis of 1H,1H,11H,11H-dodecafluoro-3,6,9-trioxaundecane bis(1,1,1,3,3,3-hexafluoroisopropyl carbonate)

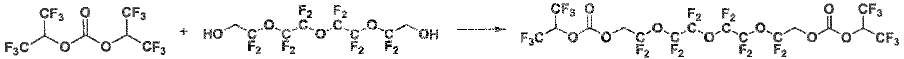

BHFC (12 mmol, 2.64 mL), 1H,1H,11H,11H-dodecafluoro-3,6,9-trioxaundecane-1,11-diol (2.0 mmol, 0.82 g), pyridine (0.20 mmol, 16 µL) and acetonitrile (1 mL) as a solvent were added into a 25 mL round-bottom flask, and the mixture was heated and stirred at 20°C for 6 hours. Then, appropriate amounts of 1 M hydrochloric acid and hydrofluoroether ("Novec^{™} 7100" manufactured by 3M) were added to the reaction mixture, and the organic phase and the aqueous phase were separated. The organic phase was dried over anhydrous sodium sulfate. The solvent was evaporated under reduced pressure from the obtained solution, and the residue was dried in vacuo at 50°C for 1.5 hours to obtain the target compound as white solid (amount: 1.49 g, 1.9 mmol, yield: 93%).
¹H NMR (400 MHz, CDCl₃, 293 K): δ5.55(sep, J=5.6Hz, 2H, CH), 4.64(t, J=8.4Hz, 4H, CH₂)
¹³C NMR (100 MHz, CDCl₃, 293 K): δ152.42, 120.04, 117.57, 114.51, 111.64, 71.24, 66.20
¹⁹F NMR (376 MHz, CDCl₃, 293 K): δ-73. 67 (m, 12F, CF₃), -77.77(m, 4F, CF₂), -88.83(m, 4F, CF₂), -88.94(s, 4F, CF₂)
IR (ATR): 2988, 2360, 1791, 1385, 1322, 1288, 1247, 1196, 1146, 1108, 1044, 990, 949, 907, 776, 690 cm⁻¹
FT-MS: m/z calculated for [M+Na]⁻ (C₁₆H₆F₂₄O₉) 820.9498, found 821.3282

### (2) Synthesis of polyurethane

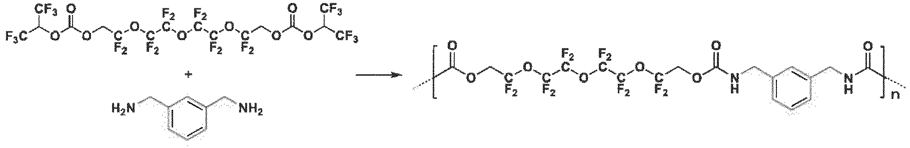

Into a 10 mL round-bottom flask, H,1H,11H,11H-dodecafluoro-3,6,9-trioxaundecane bis(1,1,1,3,3,3-hexafluoroisopropyl carbonate) (0.80 g, 1.0 mmol) and m-xylylenediamine (130 µL, 1.0 mmol) were added. The mixture was stirred at 150°C for 1 hour. Then, the insoluble precipitate was dissolved in tetrahydrofuran, and the solvent was evaporated under reduced pressure. The residue was dried in vacuo at 50°C for 2 hours to obtain the target compound as yellow solid (amount: 0.68 g, 0.99 mmol, yield: 99%).
¹H NMR (400 MHz, acetone-d₆, 293 K): δ7.30-7.21(m, 4H, CHAr), 4.66(t, J=10.2Hz, 4H, CH₂), 4.34(d, J=6.4Hz, 4H, CH₂)
¹³C NMR (125 MHz, acetone-d₆, 293 K): δ155.62, 140.25, 129.43, 127.28, 126.96, 123.15, 115.52, 113.24, 68.08, 62.29, 45.43, 26.18
¹⁹F NMR (376 MHz, acetone-d₆, 293 K): δ-78.29(m, 4F, CF₂), - 89.15(br., 4F, CF₂), -89.48(s, 4F, CF₂)
IR (ATR): 3330, 2974, 2364, 1699, 1540, 1412, 1172, 1108,
1057, 962, 774, 703 cm⁻¹
Average molecular weight by HPLC: Mₙ = 4800, M_{w} = 12100, M_{w}/Mₙ = 2.5

### Comparative example 4

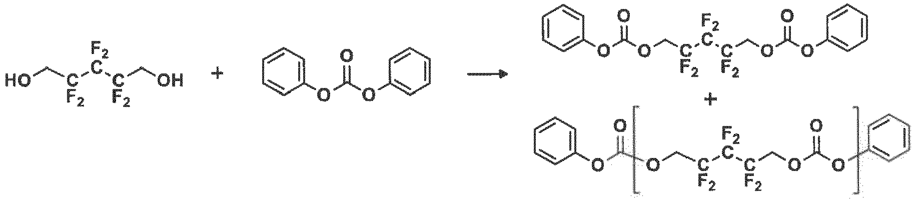

Diphenyl carbonate (24 mmol, 5.1 g), 1,5-HFPDL (4.0 mmol, 0.84 g) and pyridine (1.2 mmol, 96 µL) were added into a 50 mL round-bottom flask, and the mixture was stirred at 80°C for 12 days. Then, after the solvent was evaporated under reduced pressure, the residue was subjected to distillation under reduced pressure using a glass tube oven.

As a result, 2,2,3,3,4,4-hexafluoro-1,5-pentamethylenediphenyl carbonate was obtained as the yellow viscous solid target compound (amount: 1.1 g, 2.5 mmol, yield: 63%), but the target compound contained 62% of a polymer.

As the above-described result, when diphenyl carbonate was used in place of a fluorocarbonate compound, a polycarbonate was produced and thus the synthesis of polyurethane became difficult due to the progress of undesired polymerization reaction.
¹H NMR (400 MHz, CDCl₃, 293 K): δ7.41 (t, J=8.0Hz, 4H, CHAr), 7.30-7.26(m, 2H, CHAr), 7.21-7.18(m, 4H, CHAr), 4.79-4.65(br., 10H, CH₂+CH₂)
¹⁹F NMR (376 MHz, CDCl₃, 293 K): 5-119.98 to -120.18(m, 4F, CF₂), -125.62(m, 2F, CF₂)
IR (ATR): 1772, 1593, 1496, 1410, 1240, 1154, 984, 895, 772, 687 cm⁻¹

### Comparative example 5

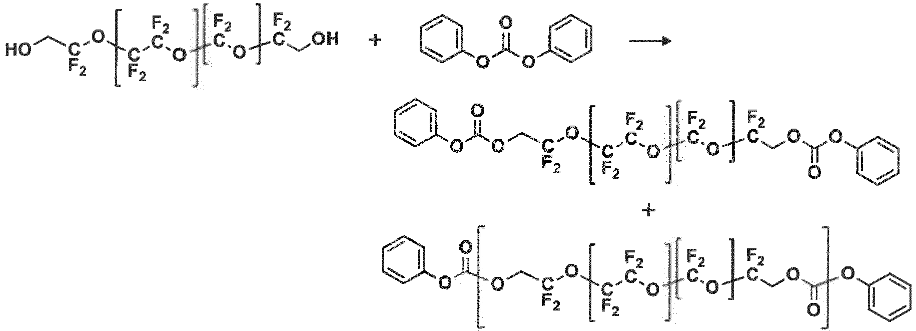

Diphenyl carbonate (10 mmol, 2.1 g), pentafluoropolyether ("Fomblin (registered trademark) D2" manufactured by Solvay) (1.0 mmol, 1.5 g) and pyridine (1.0 mmol, 81 µL) were added into a 50 mL round-bottom flask, and the mixture was violently stirred at 85°C for 9 days in order to mix the phase-separating solution. After the reaction, dichloromethane was added to the reaction mixture and the liquid part was removed by decantation. Then, distillation under reduced pressure was carried out using a glass tube oven.

As a result, perfluoropolyetherdiphenyl carbonate was obtained (amount: 0.67 g, 0.38 mmol, yield: 38%) as the colorless liquid target compound, but the target compound contained 76% of a polymer.

As the above-described result, when diphenyl carbonate is used in place of a fluorocarbonate compound, the progress of the reaction was slowed down, probably since the reaction mixture separated into two phases. In addition, a polycarbonate was produced and thus the synthesis of polyurethane became difficult due to the progress of undesired polymerization reaction.
¹H NMR (400 MHz, CDCl₃, 293 K): δ7.40(t, J=8.0Hz, 4H, CHAr), 7.30-7.26(m, 2H, CHAr), 7.18(d, J=7.6Hz, 4H, CHAr), 4.61-4.50(br., 17H, CH₂+CH₂)
¹⁹F NMR (376 MHz, CDCl₃, 293 K): δ-54.88 (m, 12F, CF₂), -80.61(m, 4F, CF₂), -89.00 to -91.92(m, 32F, CF₂)
IR (ATR): 1790, 1182, 1055, 826 cm⁻¹

## Claims

1. A method for producing polyurethane, the method comprising the steps of:
reacting a fluorocarbonate compound represented by the following formula (I) and a divalent alcohol compound represented by the following formula (II) to obtain a biscarbonate compound represented by the following formula (III), 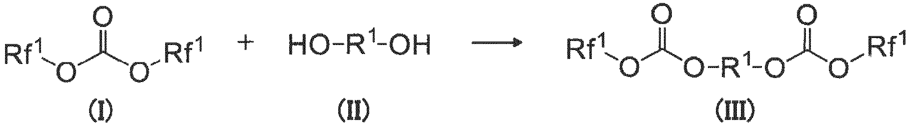 wherein
Rf¹ is independently an aliphatic hydrocarbon group having a fluoro group,
R¹ is a divalent organic group,
and reacting the biscarbonate compound represented by the above formula (III) and a divalent amino compound represented by the following formula (IV) to obtain a polyurethane represented by the following formula (V), 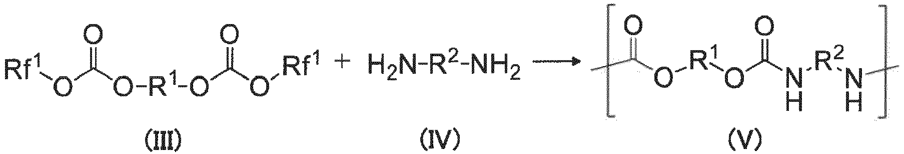 wherein
Rf¹ and R¹ are the same as the above,
R² is a divalent organic group.

2. The method according to claim 1, wherein the fluorocarbonate compound represented by the formula (I) and the divalent alcohol compound represented by the formula (II) are reacted in the presence of a base.

3. The method according to claim 1 or 2, wherein R¹ is a C₂₋₁₀ alkanediyl group optionally substituted by a halogeno group.

4. The method according to claim 1 or 2, wherein R¹ is the divalent organic group represented by the formula -R³-[-X-R³-]ₘ-, wherein X is O or S, R³ is a C₁₋₈ alkanediyl group optionally substituted by a halogeno group, and m is an integer of 1 or more and 180 or less.

5. The method according to any one of claims 1 to 4, wherein R² is a C₂₋₁₀ alkanediyl group optionally having a halogeno group.

6. The method according to any one of claims 1 to 4, wherein R² is a C₁₋₆ alkanediyl - C₆₋₁₂ aryldiyl - C₁₋₆ alkanediyl group.

7. A biscarbonate compound represented by the following formula (III-1): 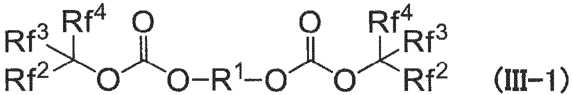 wherein
Rf² is H or an aliphatic hydrocarbon group having a fluoro group,
Rf³ and Rf⁴ are independently an aliphatic hydrocarbon group having a fluoro group,
R¹ is a divalent organic group.

## Patentansprüche

1. Verfahren zur Herstellung von Polyurethan, wobei das Verfahren die nachstehenden Schritte umfasst:
das Umsetzen einer Fluorcarbonatverbindung, dargestellt durch die nachstehende Formel (I), und einer zweiwertigen Alkoholverbindung, dargestellt durch die nachstehende Formel (II), um eine Biscarbonatverbindung, dargestellt durch die nachstehende Formel (III), zu erhalten 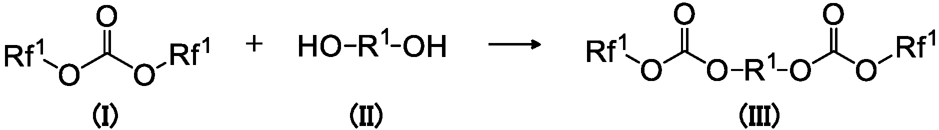 wobei
Rf¹ unabhängig eine aliphatische Kohlenwasserstoffgruppe mit einer Fluorgruppe ist,
R¹ eine zweiwertige organische Gruppe ist,
und das Umsetzen der Biscarbonatverbindung, die durch die vorstehende Formel (III) dargestellt ist, und einer zweiwertigen Aminoverbindung, die durch die nachstehende Formel (IV) dargestellt ist, um ein Polyurethan zu erhalten, das durch die nachstehende Formel (V) dargestellt ist 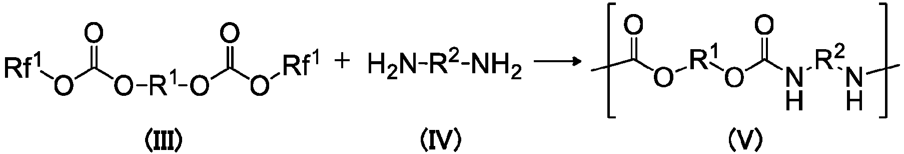 wobei
Rf¹ und R¹ die gleichen sind wie vorstehend,
R² eine zweiwertige organische Gruppe ist.

2. Verfahren nach Anspruch 1, wobei die Fluorcarbonatverbindung, dargestellt durch die Formel (I), und die zweiwertige Alkoholverbindung, dargestellt durch die Formel (II), in Gegenwart einer Base umgesetzt werden.

3. Verfahren nach Anspruch 1 oder 2, wobei R¹ eine C₂₋₁₀-Alkandiylgruppe ist, die gegebenenfalls durch eine Halogengruppe substituiert ist.

4. Verfahren nach Anspruch 1 oder 2, wobei R¹ die zweiwertige organische Gruppe ist, die durch die Formel -R³-[-X-R³-]ₘ-dargestellt ist, wobei X O oder S ist, R³ eine C₁₋₈-Alkandiylgruppe ist, die gegebenenfalls durch eine Halogengruppe substituiert ist, und m eine ganze Zahl von 1 oder mehr und 180 oder weniger ist.

5. Verfahren nach einem der Ansprüche 1 bis 4, wobei R² eine C₂₋₁₀-Alkandiylgruppe ist, die gegebenenfalls eine Halogengruppe aufweist.

6. Verfahren nach einem der Ansprüche 1 bis 4, wobei R² eine C₁₋₆-Alkandiyl-C₆₋₁₂-Aryldiyl-C₁₋₆-Alkandiylgruppe ist.

7. Biscarbonatverbindung, dargestellt durch die nachstehende Formel (III-1): 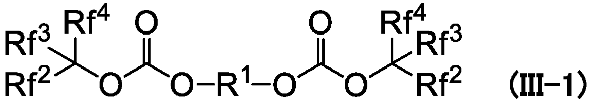 wobei
Rf² H oder eine aliphatische Kohlenwasserstoffgruppe mit einer Fluorgruppe ist,
Rf³ und Rf⁴ unabhängig eine aliphatische Kohlenwasserstoffgruppe mit einer Fluorgruppe sind,
R¹ eine zweiwertige organische Gruppe ist.

## Revendications

1. Procédé de production de polyuréthane, le procédé comprenant les étapes consistant à :
faire réagir un composé de fluorocarbonate représenté par la formule suivante (I) et un composé d'alcool divalent représenté par la formule suivante (II) pour obtenir un composé de biscarbonate représenté par la formule suivante (III), 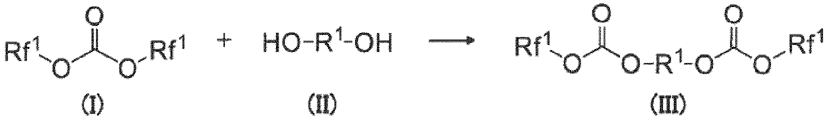
dans lesquelles
Rf¹ est indépendamment un groupe hydrocarbure aliphatique ayant un groupe fluoro,
R¹ est un groupe organique divalent,
et faire réagir le composé de biscarbonate représenté par la formule ci-dessus (III) et un composé amino divalent représenté par la formule suivante (IV) pour obtenir un polyuréthane représenté par la formule suivante (V), 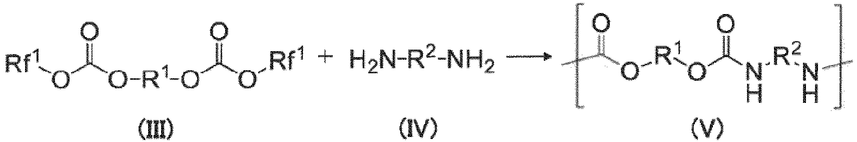
dans lesquelles
Rf¹ et R¹ sont identiques aux éléments ci-dessus,
R² est un groupe organique divalent.

2. Procédé selon la revendication 1, dans lequel le composé de fluorocarbonate représenté par la formule (I) et le composé d'alcool divalent représenté par la formule (II) sont mis à réagir en présence d'une base.

3. Procédé selon la revendication 1 ou 2, dans lequel R¹ est un groupe alcanediyle en C₂₋₁₀ substitué en option par un groupe halogéno.

4. Procédé selon la revendication 1 ou 2, dans lequel R¹ est le groupe organique divalent représenté par la formule -R³-[-X-R³-]ₘ-, dans laquelle X est O ou S, R³ est un groupe alcanediyle en C₁₋₈ substitué en option par un groupe halogéno, et m est un entier de 1 ou plus et 180 ou moins.

5. Procédé selon l'une quelconque des revendications 1 à 4, dans lequel R² est un groupe alcanediyle en C₂₋₁₀ ayant en option un groupe halogéno.

6. Procédé selon l'une quelconque des revendications 1 à 4, dans lequel R² est un groupe alcanediyle en C₁₋₆ - aryldiyle en C₆₋₁₂ - alcanediyle en C₁₋₆.

7. Composé de biscarbonate représenté par la formule suivante (III-1) : 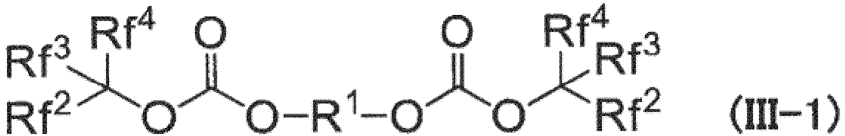
dans laquelle
Rf² est H ou un groupe hydrocarbure aliphatique ayant un groupe fluoro,
Rf³ et Rf⁴ sont indépendamment un groupe hydrocarbure aliphatique ayant un groupe fluoro,
R¹ est un groupe organique divalent.
